(19) Europäisches Patentamt
European Patent Office
Office européen des brevets

(11) Veröffentlichungsnummer: **0 045 281**
**B1**

(12) # EUROPÄISCHE PATENTSCHRIFT

(45) Veröffentlichungstag der Patentschrift: **08.05.85**

(21) Anmeldenummer: **81810299.8**

(22) Anmeldetag: **22.07.81**

(51) Int. Cl.⁴: **C 07 D 417/04,** C 07 D 417/14, A 61 K 31/44, C 12 P 17/16 // (C07D417/04, 213:65, 277:12)

(54) **Thiazolinderivate und Verfahren zu ihrer Herstellung.**

(30) Priorität: **28.07.80 CH 5755/80**
**05.08.80 CH 5925/80**

(43) Veröffentlichungstag der Anmeldung: **03.02.82 Patentblatt 82/5**

(45) Bekanntmachung des Hinweises auf die Patenterteilung: **08.05.85 Patentblatt 85/19**

(84) Benannte Vertragsstaaten: **AT BE CH DE FR IT LI LU NL SE**

(56) Entgegenhaltungen:
**DE - A - 2 053 234**
**DE - A - 2 331 246**
**DE - A - 2 729 414**
**GB - A - 1 449 669**
**US - A - 3 842 172**

(73) Patentinhaber: **CIBA-GEIGY AG, Postfach, CH-4002 Basel (CH)**

(72) Erfinder: **Zähner, Hans, Prof. Dr., Im Hopfengarten 13, D-7400 Tübingen (DE)**
Erfinder: **Naegeli, Hans-Ulrich, Dr., Pestalozzistrasse 33, CH-4132 Muttenz (CH)**
Erfinder: **Peter, Heinrich, Dr., Bündtenweg 69, CH-4102 Binningen (CH)**

**EP 0 045 281 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Derivate der 2-(Pyrid-2'-yl)-2-thiazolin-4-carbonsäure, Verfahren zu deren Herstellung, pharmazeutische Präparate, die solche Verbindungen enthalten, und die Verwendung dieser Derivate.

Stand der Technik

Die DE-AL-2 729 414 beschreibt N-acylierte Thiazolidin-4-carbonsäure-derivate, die einen unsubstituierten 3-Pyridylrest tragen, zur Auflösung von Nieren-, Leber- und Gallenblasensteinen.

Die DE-A-2 053 234 beschreibt Thiazolidinderivate der Formel

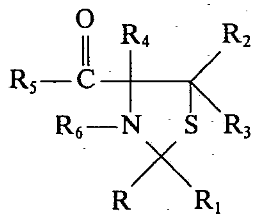

worin R, $R_1$, $R_2$, $R_3$, $R_4$ und $R_6$ jeweils Wasserstoff, Niederalkyl, Niederalkenyl, Niederalkinyl, Cycloniederalkyl, Cycloniederalkenyl, Aryl oder Heteroaryl und $R_5$ Wasserstoff, Niederalkoxy, Amino, Alkylamino oder Dialkylamino bedeuten, mit entzündungshemmender Wirkung.

Die DE-A-2 331 246 beschreibt Thiazolderivate, die einen gegebenenfalls durch Niederalkyl substituierten 3-Pyridylrest tragen, mit einer hemmenden Wirkung auf die Bildung von Granulomgewebe.

Die GB-A-1 449 669, die der US-A-3 842 172 im wesentlichen äquivalent ist, beschreibt Thiazolderivate, die in 2-Stellung durch unsubstituiertes 3-Pyridyl und in 4-Stellung durch $C_{1-4}$-Alkyl oder gegebenenfalls N-alkyliertes Carbamoyl substituiert sind und die agressivem Verhalten entgegenwirken.

Von dem obengenannten Stand der Technik unterscheiden sich die erfindungsgemäßen Verbindungen deutlich sowohl strukturell als auch hinsichtlich ihrer pharmakologischen Wirkung.

Die Erfindung betrifft 2-(3'-Substit.-pyrid-2'-yl)-2-thiazolin-4-carbonsäure-Derivate der Formel (I)

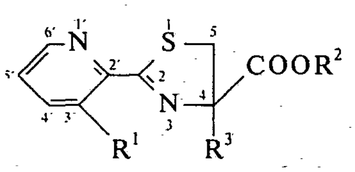

(I)

in racemischer oder optisch aktiver Form, worin $R^1$ für frei Hydroxy, Hydrocarbyloxy, Hydrocarbylcarbonyloxy oder Hydrocarbyloxycarbonyloxy steht, wobei Hydrocarbyl einen aliphatischen Rest mit 1—7 C-Atomen, einen cycloaliphatischen Rest mit 5 oder 6 Ringgliedern, einen Phenylrest oder einen durch mindestens einen solchen cycloaliphatischen Rest oder Phenylrest substituierten aliphatischen Rest mit 1—7 C-Atomen bedeutet, oder worin $R^1$ für monocyclisches fünf- oder sechsgliedriges Heterocyclyloxy mit einem Stickstoff-, Sauerstoff- oder Schwefelatom als Ringglied oder für Toluolsulfonyloxy oder aliphatisches Sulfonyloxy mit 1—7 C-Atomen steht, worin $R^2$ für Wasserstoff, einen aliphatischen Rest mit 1—7 C-Atomen, einen cycloaliphatischen Rest mit 5 oder 6 Ringgliedern, einen Phenylrest oder einen aliphatischen Rest mit 1—7 C-Atomen steht, der durch mindestens einen solchen cycloaliphatischen Rest oder Phenylrest subsituiert ist, und worin $R^3$ Wasserstoff oder $C_{1-7}$-Alkyl bedeutet, und Salze dieser Verbindungen und Eisen-, Aluminium- oder Chromkomplexe solcher Verbindungen mit freier 4-Carboxy-Gruppe, Verfahren zur Herstellung dieser Verbindungen, solche Verbindungen enthaltende pharmazeutische Mittel und die Verwendung dieser Verbindungen.

Aliphatisches Hydrocarbyl in einem Hydrocarbyloxy-, Hydrocarbylcarbonyloxy- oder Hydrocarbyloxycarbonyloxyrest $R^1$ hat 1—7, vorzugsweise 1—4, C-Atome und steht vornehmlich für entsprechendes unsubstituiertes oder substituiertes Alkoxy, in allererster Linie für unsubstituiertes Alkoxy, z. B. Methoxy.

Ein cycloaliphatischer Rest mit 5 oder 6 Ringgliedern in einem Hydrocarbyloxy-, Hydrocarbylcarbonyloxy- oder Hydrocarbyloxycarbonyloxyrest $R^1$ ist vor allem ein solcher unsubstituierter oder substituierter Cycloalkylrest, insbesondere ein unsubstituierter oder durch Niederalkyl substituierter Cycloalkylrest. Ein Phenylrest in einem Hydrocarbyloxy-, Hydrocarbylcarbonyloxy- oder Hydrocarbyloxycarbonyloxyrest $R^1$ ist in allererster Linie Phenyl.

Monocyclisches fünf oder sechsgliedriges Heterocyclyl mit einem Stickstoff-, Sauerstoff oder Schwefelatom als Ringglied in einem Heterocyclyloxyrest $R^1$ steht vorzugsweise für derartiges unsubstituiertes oder substituiertes, gesättigtes oder ungesättigtes, insbesondere für gesättigtes, Heterocyclyl, vor allem für gesättigtes und unsubstituiertes Heterocyclyl, dessen freie Valenz sich in Nachbarschaft zum Heteroatom befindet, z. B. 2-Tetrahydropyranyl.

Aliphatisches Sulfonyloxy $R^1$ hat 1—7, in erster Linie 1—4 C-Atome und ist insbesondere unsubstituiertes oder halogeniertes Alkylsulfonyloxy.

Ein aliphatischer Rest $R^2$ hat 1—7, vorzugsweise 1—4 C-Atome und steht vornehmlich für entsprechendes unsubstituiertes oder substituiertes Alkyl, in allererster Linie für unsubstituiertes Alkyl, z. B. Methyl.

Ein cycloaliphatischer Rest $R^2$ mit 5 oder 6 Ringgliedern ist vor allem ein solcher unsubstituierter oder substituierter Cycloalkylrest, insbesondere ein unsubstituierter oder durch Niederalkyl substituierter Cycloalkylrest. Ein Phenylrest $R^2$ ist in allererster Linie Phenyl.

Ein aliphatischer Rest $R^2$, der durch mindestens einen wie oben definierten Phenylrest substituiert ist, ist z. B. Benzyl.

$C_{1-7}$-Alkyl $R^3$ ist in allererster Linie Methyl.

Die vor- und/oder nachstehend verwendeten Allgemeindefinitionen haben im Rahmen der vorliegenden Beschreibung vorzugsweise die folgenden Bedeutungen:

»Nieder-« umschreibt einen Rest mit 1—7 C-Atomen. Niederalkyl ist z. B. Methyl, Ethyl, n-Propyl, Isopropyl, n-Butyl, Isobutyl, sek.-Butyl oder tert.-Butyl, ferner n-Pentyl, Neopentyl, n-Hexyl oder n-Heptyl. Cycloalkyl mit 5 oder 6 Ringgliedern ist z. B. Cyclopentyl oder Cyclohexyl. Phenylniederalkyl ist z. B. Benzyl, 1- oder 2-Phenylethyl oder 3-Phenylpropyl. Halogen ist insbesondere Brom, kann jedoch auch für Chlor oder Jod, ferner für Fluor stehen. Niederalkoxy ist z. B. Methoxy, ferner Ethoxy, n-Propoxy, Isopropoxy, n-Butoxy, Isobutoxy oder tert.-Butoxy. Niederalkanoyloxy ist z. B. Acetoxy oder Propionyloxy. Niederalkoxycarbonyl ist z. B. Methoxycarbonyl oder Ethocycarbonyl. Niederalkoxycarbonyloxy ist z. B. Methoxycarbonyloxy oder Ethoxycarbonyloxy. Fünf- oder sechsgliedriges Heteroxyclyl mit einem Sauerstoff-, Stickstoff- oder Schwefelatom als Ringglied ist z. B. Tetrahydropyranyl, Pyridyl, Thienyl oder Furyl.

Substituenten von Phenyl, sind gegebenenfalls substituiertes Niederalkyl oder gegebenenfalls, z. B. durch Niederalkyl, Nitro-, Niederalkoxy und/oder Halogen, substituiertes Phenyl oder funktionelle Gruppen, z. B. basische Gruppen, wie eine gegebenenfalls substituierte Aminogruppe, oder saure Gruppen, wie gegebenenfalls funktionell abgewandeltes, z. B. verestertes Carboxy, z. B. Carboxy oder Niederalkoxycarbonyl, oder gegebenenfalls verethertes, verestertes oder geschütztes Hydroxy oder Oxo.

Niederaliphatisches Sulfonyloxy ist z. B. Methansulfonyloxy.

Schutzgruppen, beispielsweise für die 3'-Hydroxy-Gruppe, sowie ihre Einführung und Abspaltung sind beispielsweise beschrieben in »Protective Groups in Organic Chemistry«, Plenum Press, London, New York 1973, ferner in »Methoden der organischen Chemie«, Houben-Weyl, 4. Auflage, Bd. 15/1, Georg Thieme Verlag, Stuttgart 1974. Charakteristisch für Schutzgruppen ist, daß sie leicht, d. h. ohne daß unerwünschte Nebenreaktionen stattfinden, z. B. solvolytisch, reduktiv, photolytisch oder auch unter physiologischen Bedingungen abspaltbar sind.

Hydroxyschutzgruppen sind z. B. Acylreste, wie gegebenenfalls, z. B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z. B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z. B. tert.-Butyl, 2-oxy- oder 2-thia-aliphatische oder -cycloaliphatische Kohlenwasserstoffreste, in erster Linie 1-Niederalkoxynideralkyl oder 1-Niederalkylthio-niederalkyl, z. B. Methoxymethyl, 1-Methoxyethyl, 1-Ethoxy-ethyl, 1-Methylthiomethyl, 1-Methylthiomethyl oder 1-Ethylthioethyl, oder 2-Oxa- oder 2-Thiacycloalkyl mit 5—6 Ringatomen, z. B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z. B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen, wie den Rest $R^2$, in erster Line in 1-Stellung verzweigte oder in 1- oder 2-Stellung geeignet substituierte Niederalkylgruppen. Bevorzugte, in veresterter Form vorliegende Carboxylgruppen sind u. a. tert.-Niederalkoxycarbonyl, z. B. tert.-Butyloxycarbonyl, Arylmethoxycarbonyl, mit einem oder zwei Arylresten, wobei dieses gegebenenfalls z. B. durch Niederalkyl, wie tert.-Niederalkyl, z. B. tert.-Butyl, Niederalkoxy, wie Methoxy, Hydroxy, Halogen, z. B. Chlor, und/oder Nitro, mono- oder polysubstituierte Phenylreste darstellen, wie gegebenenfalls, z. B. wie oben erwähnt, substituiertes Benzyloxycarbonyl, z. B. 4-Nitro-benzyloxycarbonyl oder 4-Methoxybenzyloxycarbonyl, oder gegebenenfalls, z. B. wie oben erwähnt, substituiertes Diphenylmethoxycarbonyl, z. B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z. B. durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Jodethoxycarbonyl, oder 2-(trisubstituiertes Silyl)-ethoxycarbonyl, worin die Substituenten unabhängig voneinder je einen gegebenenfalls substituierten, z. B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z. B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z. B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z. B. wie oben erwähnt, substituiertes Benzyloxycarbonyl, wie 4-Nitro-benzyloxycarbonyl, oder Diphenylmethoxycarbonyl, vor allem 2-(Trimethylsilyl)-ethoxycarbonyl.

Die Salze der erfindungsgemäßen Verbindungen sind in erster Linie pharmazeutisch verwendbare, nicht-toxische Salze, wie diejenigen von Verbindungen der Formel (I) mit sauren Gruppen, z. B. mit einer freien Carboxylgruppe. Solche Salze sind in erster Linie innere Salze, wobei eine im Molekül vorhandene basische Gruppe, z. B. ein basisches Stickstoffatom, z. B. im Pyridinring oder in einer gegebenenfalls vorhandenen freien Aminogruppe, durch ein aus einem Molekül der Formel (I) stammendes Wasserstoffion protoniert ist, daneben auch Metall- oder Ammoniumsalze, wie Alkalimetall- oder Erdalkalimetall-, z. B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z. B. Triethylamin, Hydroxyniederalkylamine, z. B. 2-Hydroxyethylamin, Bis-(2-hydroxyethyl)-amin oder Tris-(2-hydroxyethyl)-amin, basische aliphatische Ester von Carbonsäuren, z. B. 4-Aminobenzoesäure-2-diethylaminoethylester, Niederalkylenamine, z. B. 1-Ethyl-piperidin, Cycloalkylamine, z. B. Dicyclohexylamin, oder Benzylamine, z. B. N,N'-Dibenzyl-ethylendiamin, ferner Basen vom Pyridintyp, z. B. Pyridin, Collidin oder Chinolin. Die Verbindungen der Formel (I) können auch intermolekulare (im Gegensatz zu den intramolekularen, d. h. zwitterionischen) Säureadditionssalze, z. B. mit anorganischen Säuren, wie Salzsäure, Schwefelsäure oder Phosphorsäure, oder mit geeigneten organischen Carbon- oder Sulfonsäure, z. B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Bei Verbindungen der Formel (I) mit einer sauren Gruppe, z. B. einer freien Carboxylgruppe, kann auch ein Teil des Moleküls als inneres Salz und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Die Verbindungen der Formel (I) mit freier Carboxylgruppe, wobei das Proton der Carboxylgruppe abgespalten sein kann, vermögen mit Metallionen, wie Schwermetallionen, stabile Komplexe zu bilden. Unter den Schwermetallionen sind insbesondere diejenigen mit der Ionenwertigkeit $3^{+}$ zu nennen, wie $Al^{3+}$ oder $Cr^{3+}$, in allererster Linie aber $Fe^{3+}$.

Zugabe der 2-(3'-Hydroxy-pyrid-2'-yl)-4-methyl-2-thiazolin-4-carbonsäure zu einem Eisen(III)-Ethylendiamintetraessigsäure-dinatrium-Salz-Komplex ($\log K = 21{,}6$) führt zu einer Umkomplexierung, aus deren polarographisch verfolgtem Verlauf sich der dekadische Logarithmus log K der Komplexbildungskonstanten K des 2-(3'-Hydroxy-pyrid-2'-yl)-4-methyl-2-thiazolin-4-carbonsäure-Eisen(III)-Komplexes (Ferrithiocin) zu 32,7 und seine Ligandzahl p zu 3 ergibt.

Die Substanzen der Formel (I) besitzen pharmakologisch wertvolle Eigenschaften. Insbesondere sind die Verbindungen der Formel (I) antibakteriell wirksam und können daher in Form pharmazeutischer Präparate, z. B. zur Behandlung von Infektionen verwendet werden.

Eine 1%ige Lösung der 2-(3'-Hydroxy-pyrid-2'-yl)-4-methyl-2-thiazolin-4-carbonsäure in Wasser wurde im Agar-Diffusionstest gegen die folgenden Bakterien auf antibiotische Wirkung untersucht, wobei die in Klammern angegebenen Hemmzonen [mm] beobachtet wurden:

Sarcina lutea (13), Bac. subtilis (auf synth. Medium, 25), E. coli, supersensitiv $EV_2$ (18), Proteus mirabilis (13), Proteus vulgaris (15) und Xanthomonas oryzae (22).

Aufgrund ihrer Eigenschaften mit Schwermetallionen, besonders mit solchem mit der Ionenwertigkeit 3+, wie $Al^{3+}$ oder $Cr^{3+}$, vor allem aber mit $Fe^{3+}$, stabile Komplexe zu bilden, verhindern die Verbindungen der Formel (I) mit freier 4-Carboxy-Gruppe, z. B. 2-(3'-Hydroxy-pyrid-2'-yl)-4-methyl-2-thiazolin-carbonsäure, z. B. die Ablagerung eisenhaltiger Pigmente im Gewebe bzw. sie bewirken in Fällen von Eisenablagerung im Organismus eine Ausscheidung des Eisens, z. B. bei Hämochromatose und Hämosiderose sowie auch bei Lebercirrhose. Sie können auch zur Ausscheidung anderer Schwermetalle, z. B. der obengenannten oder von Kupfer, aus dem Organismus verwendet werden. Beispielsweise bewirkt die subcutane Applikation von 10 mg/kg 2-(3'-Hydroxy-pyrid-2'-yl)-4-methyl-2-thiazolin-4-carbonsäure bei der Ratte ein im Urin nachweisbare Ausscheidung von Ferrithiocin. Die therapeutische Dosis der letztgenannten Substanz als Eisenentzugsmittel beträgt bei Warmblütern von etwa 70 kg Körpergewicht, z. B. beim Menschen, 100—1000 mg, insbesondere 300—700 mg, z. B. 500 mg, täglich. Vorzugsweise appliziert man die Dosis verteilt auf mehrere z. B. drei, Einzelgaben z. B. oral, aber auch subcutan.

Zum Zwecke der Ausscheidung von Schwermetallionen, z. B. von Eisen(III)ionen, kann man auch Verbindungen der Formel (I) mit veresterter 4-Carboxy-Gruppe verwenden, deren Estergruppierung unter physiologischen Bedingungen leicht verseift wird (Prodrug-Formen).

Auf der anderen Seite können die eisenhaltigen Komplexe in bestimmten Fällen als Eisendonatoren, z. B. bei Nutztieren, zur Anwendung gelangen.

Beispielsweise kann der Eisenkomplex der 2-(3'-Hydroxy-pyrid-2'-yl)-4-metyl-2-thiazolin-4-carbonsäure zur Prophylaxe und Therapie der Eisenmangelanämie bei Saugferkeln eingesetzt werden. Zu diesem Zweck werden zwei Dosierungen, welche einem Eisengehalt von zusammen 100 mg pro Ferkel entsprechen, nach 11 und 16 Lebenstagen in Form einer feinverteilten Suspension in 40 ml 0,6 m Phosphatpuffer pH 7,4 mit einer Gummisonde direkt in den Magen appliziert. Das Gewicht der Ferkel beträgt bei der Geburt durchschnittlich 1,7 kg, nach 11 Lebenstagen 3—4 kg, nach 16 Lebenstagen 3,7—5,1 kg und nach 22 Lebenstagen 4,5 bis 6,5 kg. Die Messung der Hämoglobinkonzentration nach 11 Versuchstagen (22 Lebenstagen) ergibt im Vergleich zu einer Negativkontrolle (entsprechende Pufferlösung ohne Wirkstoff) signifikant erhöhte Werte.

Bei der Prüfung auf akute Toxizität an der Maus (5 Tiere von 20—25 g) erwies sich die 2-(3'-Hydroxy-pyrid-2'-yl)-4-methyl-2-thiazolin-4-carbonsäure bei einer Dosis von 100 mg/kg sowohl bei peroraler, wie auch bei subkutaner Applikation als voll verträglich.

Verbindungen der Formel (I), worin die funktionellen Gruppen geschützt sind, werden in erster Linie als Zwischenprodukte zur Herstellung solcher Verbindungen der Formel (I) verwendet, worin die funktionellen Gruppen in freier Form vorliegen.

In erster Linie betrifft die Erfindung Verbindungen der Formel (I), worin $R^1$ freies Hydroxy, Alkoxy mit 1—7 C-Atomen, Toluolsufonyloxy, Niederalkylsulfonyloxy, Hydrocarbylcarbonyloxy oder Hydrocarbyloxycarbonyloxy bedeutet, wobei Hydrocarbyl für Niederalkyl, Cycloalkyl mit 5 oder 6 C-Atomen, Phenyl oder für Phenylniederalkyl steht, und worin $R^2$ einen solchen Hydrocarbylrest oder Wasserstoff und $R^3$ Wasserstoff oder Niederalkyl bedeutet.

Bevorzugterweise betrifft die Erfindung Verbindungen der Formel (I), worin $R^1$ für freies Hydroxy, Niederalkoxy, Niederalkanoyloxy oder für jeweils unsubstituiertes oder durch Halogen, Niederalkyl oder Niederalkoxy substituiertes Phenyloxy oder Benzyloxy steht, und worin $R^2$ Wasserstoff, Niederalkyl oder einen Phenyl- oder Phenylniederalkylrest und $R^3$ Wasserstoff oder Niederalkyl bedeuten.

Besonders bevorzugt sind Verbindungen der Formel (I), worin $R^1$ für freies Hydroxy oder Niederalkoxy, $R^2$ für Wasserstoff oder Niederalkyl und $R^3$ für Methyl stehen, und Salze dieser Verbindungen sowie Eisenionkomplexe solcher Verbindungen mit freier 4-Carboxy-Gruppe.

Hervorzuheben sind die Verbindungen der Formel (I), worin $R^1$ für Hydroxy oder Methoxy, $R^2$ für Wasserstoff oder Methyl und $R^3$ für Wasserstoff oder Methyl stehen, sowie deren Salze.

Besonders hervorzugeben sind die vorstehend genannten Verbindungen der Formel (I), worin $R^2$ für Wasserstoff steht, und deren Salze, in erster Linie pharmazeutisch verwendbare Salze sowie Eisenkomplexe, besonders Eisen(III)-komplexe.

Ganz besonders bevorzugt sind die Verbindungen der Formel (I), worin $R^3$ für Methyl steht, und deren Salze.

Die Erfindung betrifft insbesondere die in den Beispielen beschriebenen Verbindungen der Formel (I), sowie Salze, besonders pharmazeutisch verwendbare Salze, in allererster Linie 2-(3'-Hydroxypyrid-2'-yl)-4-methyl-2-thiazolin-4-carbonsäure, vornehmlich in der nach Beispiel 2 erhältlichen optisch aktiven Form.

Die erfindungsgemäßen Verbindungen der Formel (I) und Salze dieser Verbindungen sowie Eisen-, Aluminium- oder Chromkomplexe von solchen Verbindungen mit freier 4-Carboxy-Gruppe können durch chemische Synthese nach an sich bekannten Verfahren hergestellt werden. Sie werden beispielsweise hergestellt, indem man ein Picolinsäurederivat der Formel (II);

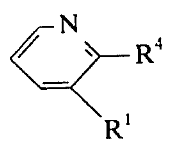

(II)

worin $R^1$ die obengenannte Bedeutung hat mit der Maßgabe, daß Hydroxy- oder andere funktionelle Gruppen gegebenenfalls in geschützter Form vorliegen, und $R^4$ eine Cyano- oder Carboxygruppe oder ein reaktives funktionelles Derivat derselben bedeuten, mit einem 2-Amino-3-mercapto-2-$R^3$-propionsäurederivat der Formel (III),

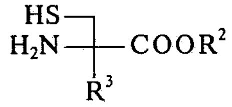

(III)

worin die Substituenten wie oben definiert sind, mit der Maßgabe, daß eine oder mehrere in der Verbindung vorhandene funktionelle Gruppen gegebenenfalls in geschützer Form vorliegen, oder mit einem reaktiven funktionellen Derivat davon umsetzt und, wenn erwünscht, gegebenenfalls vorhandene Schutzgruppen abspaltet und, wenn erwünscht, eine Verbindung der Formel (I) dadurch in eine andere Verbindung der Formel (I) überführt, daß man in einer erhaltenen Verbindung der Formel (I) eine 3'-Hydroxy-Gruppe verestert oder verethert oder eine veresterte oder veretherte 3'-Hydroxy-Gruppe in eine frei überführt und/oder eine 4-Carboxy-Gruppe mit einem den Rest $R^2$ enthaltenden Veresterungsmittel verestert oder eine veresterte 4-Carboxy-Gruppe verseift und/oder in Verbindungen der Formel (I) in ihre Säureadditionssalze oder die Verbindungen der Formel (I) mit freier 4-Carboxy-Gruppe in ihre Metallsalze oder in die Eisen-, Aluminium- oder Chromkomplexe überführt oder Racemate in die optisch aktiven Formen auftrennt.

Ein reaktives funktionelles Derivat einer Carboxylgruppe $R^4$ ist beispielsweise ein Säureanhydrid, ein aktivierter Ester oder ein aktiviertes Amid.

Unter den Anhydriden sind die gemischten Anhydride besonders geeignet. Gemischte Anhydride sind z. B. diejenigen mit anorganischen Säuren, wie Halogenwasserstoffsäuren, d. h. die entsprechenden Säurehalogenide, z. B. -chloride oder -bromide, ferner mit Stickstoffwasserstoffsäure, d. h. die entsprechenden Säureazide, mit einer phosphorhaltigen Säure, z. B. Phosphorsäure, Diäthylphosphorsäure und phosphoriger Säure, oder mit einer schwefelhaltigen Säure, z. B. Schwefelsäure, oder mit Cyanwasserstoffsäure. Weitere gemischte Anhydride sind z. B. diejenigen mit organischen Carbonsäuren, wie mit gegebenenfalls, z. B. durch Halogen, wie Fluor oder Chlor, substituierten Niederalkancarbonsäuren, z. B. Pivalinsäure oder Trichloressigsäure, oder mit Halbestern, insbesondere Niederalkylhalbestern der Kohlensäure, wie dem Äthyl- oder Isobutylhalbester der Kohlensäure, oder mit organischen, insbesondere aliphatischen oder aromatischen, Sulfonsäuren, z. B. p-Toluolsulfonsäure.

Unter den aktivierten Estern sind z. B. zu nennen: Ester mit vinylogen Alkoholen (d. h. Enolen, wie vinylogen Niederalkenolen), oder Iminomethylesterhalogeniden, wie Dimethyliminomethylesterchlorid (hergestellt aus der Carbonsäure und z. B. Dimethyl-(1-chlor-äthyliden)-iminium-chlorid der Formel

$$(CH_3)_2N^{\oplus} = C(Cl)CH_3\ Cl^{\ominus}$$

das man z. B. aus N,N-Dimethyl-acetamid und Phosgen erhalten kann), oder Arylester, wie vorzugsweise geeignete, z. B. durch Halogen, wie Chlor, und/oder Nitro, substituierte Phenylester, z. B. 4-Nitro-phenyl, 2,3-Dinitrophenyl- oder 2,3,4,5,6-Pentachlor-phenylester, N-heteroaromatische Ester, wie N-Benttriazol-, z. B. 1-Benztriazolester, oder N-Diacyliminoester, wie N-Succinylimino- oder N-Phthalyliminoester.

Geeignete aktivierte Amide sind z. B. insbesondere Imidazolide, ferner 1,2,4-Triazolide, Tetrazolide oder 1,2,4-Oxadiazolinonide.

Die Aktivierung einer Carboxylgruppe $R^4$ in dem Picolinsäurederivat kann auch in situ erfolgen.

Ein reaktives funktionelles Derivat eines 2-Amino-3-mercapto-2-$R^3$-propionsäurederivates der Formel (III) ist eine Verbindung, in der die Amino- und/oder Mercaptogruppe für die Reaktion mit der Carboxylgruppe einer Verbindung der Formel (II) aktiviert ist, also in nucleophiler Form vorliegt. Die Aminogruppe ist beispielsweise durch Reaktion mit einem Phosphit aktiviert.

Eine bevorzugte Ausführungsform der erfindungsgemäßen Reaktion ist die Umsetzung einer Verbindung der Formel (II), worin $R^4$ Cyno bedeutet, mit einem Cysteinderivat der Formel (III), die z. B. wie im Beispielteil beschrieben durchgeführt wird.

Die Reaktion von freiem Carboxyl $R^4$ mit dem gewünschten Cysteinderivat wird bevorzugterweise in Gegenwart eines geeigneten Kondensationsmittels oder unter wasserentziehenden Bedingungen, z. B. Entzug des Reaktionswassers durch azeotrope Destillation durchgeführt. Übliche Kondensationsmittel sind z. B. Carbodiimide, beispielsweise N,N'-Diäthyl-, N,N'-Dipropyl-, N,N'-Dicyclohexyl- oder N-Äthyl-N'-(3-dimethylaminopropyl)-carbodiimid, geeignete Carbonylverbindungen, beispielsweise Carbonyldiimidazol, oder 1,2-Oxazoliumverbindungen, z. B. 2-Äthyl-5-phenyl-1,2-oxazolium-3'-sulfonat und 2-tert.-Butyl-5-methyl-isoxazolium-perchlorat, oder eine geeignete Acylaminoverbindung, z. B. 2-Äthoxy-1-äthoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z. B. Methylenchlorid, Benzol oder Tetrahydrofuran und, wenn notwendig, unter Kühlen oder Erwärmen und/oder in einer Inertgasatmosphäre durchgeführt.

Verbindungen der Formel (I), worin $R^3$ Niederalkyl bedeutet, werden in an sich bekannter Weise hergestellt, indem man in einer Verbindung der Formel (I), worin $R^3$ für Wasserstoff steht und worin $R^1$ und $R^2$ wie für Formel (I) definiert sind mit der Maßgabe, daß darin vorhandene funktionelle Gruppen, wenn erwünscht, in geschützter Form vorliegen, mit einer Base in 4-Stellung ein Proton entfernt und das erhaltenen Zwischenprodukt mit einem den Rest $R^3$ übertragenden Alkylierungsmittel umsetzt, in

dem das Alkylkohlenstoffatom, das die neue Bindung eingeht, eine positive Partialladung trägt, und wenn erwünscht, gegebenenfalls vorhandene Schutzgruppen abspaltet und, wenn erwünscht, eine Verbindung der Formel (I) dadurch in eine andere Verbindung der Formel (I) überführt, daß man in einer erhaltenen Verbindung der Formel (I) eine 3'-Hydroxy-Gruppe verestert oder verethert oder eine veresterte oder veretherte 3'-Hydroxy-Gruppe in eine freie überführt und/oder eine 4-Carboxy-Gruppe mit einem den Rest $R^2$ enthaltende Veresterungsmittel verestert oder eine veresterte 4-Carboxy-Gruppe verseift und/oder die Verbindung der Formel (I) in ihre Säureadditionssalze oder die Verbindungen der Formel (I) mit freier 4-Carboxy-Gruppe in ihre Metallsalze oder in die Eisen-, Aluminium- oder Chromkomplexe überführt oder Racemate in die optisch aktiven Formen auftrennt.

Geeignete Basen sind z. B. Metallierungsreagentien, wie unsubstituierte oder substituierte Alkalimetallamide, z. B. Natriumamid, Lithiumamid oder bevorzugterweise sterisch gehinderte Lithiumamide, z. B. Lithium-trimethylsilyl-amid, Lithium-isopropyl-cyclohexyl-amid oder Lithiumdialkylamide, wie Lithiumdiisopropylamid, oder Alkalimetallniederalkylverbindungen, wie Methyl-, n- oder tert.-Butyllithium, daneben auch Alkalimetallhydride, wie Natriumhydrid.

Wenn die Reaktion unter Phasentransferbedingungen oder in Gegenwart von geeigneten Komplexbildern für das Basenkation, wie Kronenäthern, z. B. 18-Krone-6, durchgeführt wird, sind auch schwächere Basen, wie Alkalimetallhydroxide, z. B. Kaliumhydroxid, geeignet.

Geeignete den Rest $R^3$ übertragende Alkylierungsmittel sind z. B. Niederalkyl- oder Niederalkenylverbindungen mit einer nucleophilen Abgangsgruppe, z. B. Halogenide, wie Chloride, Bromide oder vor allem Jodide, z. B. Methyljodid oder Allylbromid, oder Sulfonsäure- oder Schwefelsäureester von unsubstituierten aliphatischen Alkoholen mit 1—7 C-Atomen, z. B. Toluolsulfonsäureisopropylester, Methylsulfonsäurebutylester oder Diethylsulfat.

Die mit den obengenannten Alkalimetallamiden, -hydriden oder -niederalkylverbindungen durchgeführte Metallierung wird in einem inerten, aprotischen Lösungsmittel und unter Schutzgas durchgeführt, beispielsweise in einem Kohlenwasserstoff, z. B. Hexan, Benzol, Toluol oder Xylol, einem schwach polaren Äther, z. B. Diethyläther, Tetrahydrofuran oder Dioxan, oder einem Säureamid,z. B. Hexamethylphosphorsäuretriamid, oder Mischungen davon. Die Reaktionstemperatur liegt zwischen etwa —80° und etwa Raumtemperatur, in Abhängigkeit vornehmlich vom Schmelzpunkt des Lösungsmittels oder Lösungsmittelgemisches und von der Reaktivität des jeweiligen Metallierungsreagenz in dem gewählten Lösungsmittel, aber auch von der Löslichkeit und der Reaktivität des Substrats.

Die Alkylierung erfolgt bei weit höherer Temperatur als die Metallierung und wird in der Regel so durchgeführt, daß man das Alkylierungsmittel nach beendeter Metallierung in das Reaktionsgefäß gibt, und dieses aufwärmen läßt.

Weitere Einzelheiten zur Durchführung von Metallierungs- und Alkylierungsreaktionen, die den oben beschriebenen analog sind, sind z. B. beschrieben in Houben-Weyl, Methoden der Organischen Chemie, Band XIII/1, Thieme Verlag, Stuttgart 1970.

Ein weiteres Verfahren zur Herstellung von Verbindungen der Formel (I), worin $R^1$ für eine freie Hydroxygruppe und/oder $R^2$ für Wasserstoff steht, ist dadurch gekennzeichnet, daß man in einer Verbindung der Formel (I), worin $R^1$ eine geschützte Hydroxygruppe und/oder $COOR^2$ eine geschützte Carboxylgruppe bedeuten, mindestens eine Schutzgruppe abspaltet. Die erhaltene Verbindung der Formel (I) wird, wenn erwünscht, dadurch in eine andere Verbindung der Formel (I) überführt, daß man in einer Verbindung der Formel (I) eine 3'-Hydroxy-Gruppe verestert oder verethert und/oder eine 4-Carboxy-Gruppe mit einem den Rest $R^2$ enthaltenden Veresterungsmittel verestert und/oder die Verbindungen der Formel (I) in ihre Säureadditionssalze oder die Verbindungen der Formel (I) mit freier 4-Carboxy-Gruppe in ihre Metallsalze oder in die Eisen-, Aluminium- oder Chromkomplexe überführt oder racemische Gemische in die optisch aktiven Formen auftrennt.

Verbindungen der Formel (I), worin $R^3$ eine Methylgruppe bedeutet, und die übrigen Substituenten die für Formel (I) angegebene Bedeutung haben, und die obengenannten Salze und Metallkomplexe dieser Verbindungen kann man außer nach den vorstehend beschriebenen Analogieverfahren auch nach einem neuen erfinderischen Verfahren herstellen, das dadurch gekennzeichnet ist, daß man den Stamm Streptomyces antibioticus Waksman et Henrici Tü 1998 (DSM 1965) oder eine 2-(3'-Hydroxypyrid-2'-yl)-4-methyl-2-thiazolin-4-carbonsäure bildende Mutante dieses Stammes in einer wässerigen, eine Kohlenstoff- und Stickstoffquelle sowie anorganische Salze enthaltenden Nährlösung aerob züchtet und aus der Nährlösung die 2-(3'-Hydroxy-pyrid-2'-yl)-4-methyl-2-thiazolin-4-carbonsäure oder einen stabilen Schwermetallkomplex dieser Säure isoliert und, wenn erwünscht, aus dem Schwermetallkomplex die 2-(3'-Hydroxy-pyrid-2'-yl)-4-methyl-2-thiazolin-4-carbonsäure oder ein Salz derselben freisetzt, und, wenn erwünscht, die erhaltene Verbindung dadurch in eine andere Verbindung der Formel (I) überführt, daß man die 3'-Hydroxy-Gruppe verestert oder verethert und/oder die 4-Carboxy-Gruppe mit einem den Rest $R^2$ enthaltenden Veresterungsmittel verestert und/oder die Verbindungen der Formel (I) in ihre Säureadditionssalze oder die Verbindungen der Formel (I) mit freier 4-Carboxy-Gruppe in ihre Metallsalze oder in die Eisen-, Aluminium- oder Chromkomplexe überführt.

Der Stamm Streptomyces antibioticus Tü 1998 ist unter der Bezeichnung DSM 1865 am 16. Juni 1980 bei der Deutschen Sammlung von Mikroorganismen, Grisebachstraße 8, D-3400 Göttingen, hinterlegt worden.

Der Stamm ist durch folgende Merkmale charakterisiert:

Seine Sporen sind ellipsoid, 0,6—1,4 µm × 0,5—1,2 µm groß, und haben eine glatte bis leicht warzige (event. Folge der Präparation) Oberfläche. Das Luftmycel ist anfänglich weißgrau und wird mit zunehmender Reife grau bis aschgrau.

Die Sporenketten sind monopodial verzweigt und nur leicht gewellt. Auf peptonhaltigen Nährböden, vor allem dem »pepton- iron-agar« bildet der Stamm ein dunkles melaninartiges Pigment. Das Substratmycel ist dunkel bis braunrot. Durch die auf einigen Nährböden gleichzeitig gebildeten Rubromycine erscheint das Substrat weinrot bis braunrot verfärbt.

Das Antibiotikum bildende Mutanten können zum Beispiel unter der Einwirkung von Ultraviolett- oder Röntgenstrahlen oder von chemischen Mutagenen, z. B. N-Methyl-N'-nitro-N-nitroso-guanidin, gewonnen werden.

Als Kohlenstoffquelle sind beispielsweise zu nennen: assimilierbare Kohlenhydrate, zum Beispiel Glucose, Saccharose, Lactose, Mannit, Stärke, Glycerin. Als stickstoffhaltige Nährstoffe seien genannt: Aminosäuren, Peptide und Proteine sowie deren Abbauprodukte wie Pepton oder Trypton, ferner Fleischextrakte, wasserlösliche Anteile von Getreidekörnern, wie Mais und Weizen, von Destillationsrückständen der Alkoholherstellung, von Hefe, Bohnen, insbesondere der Sojapflanze, von Samen, beispielsweise der Baumwollpflanze usw., aber auch Ammoniumsalze und Nitrate. Von anderen anorganischen Salzen kann die Nährlösung beispielsweise Chloride, Carbonate, Sulfate, Phosphate von Alkali- oder Erdalkalimetallen, von Magnesium, Eisen, Zink und Mangan enthalten.

Die Züchtung erfolgt aerob, also beispielsweise in ruhender Oberflächenkultur oder vorzugsweise submers unter Schütteln oder Rühren mit Luft oder Sauerstoff im Schüttelkolben oder den bekannten Fermentern. Als Temperatur eignet sich eine solche zwischen 18 und 40° C, vorzugsweise etwa 27° C. Eine wesentliche antibiotische Wirkung zeigt die Nährlösung dabei im allgemeinen nach 3 bis 7 Tagen. Vorzugsweise kultiviert man in mehreren Stufen, d. h. man stellt zunächst eine oder mehrere Vorkulturen in flüssigem Nährmedium her, die dann in das eigentliche Produktionsmedium, zum Beispiel im Verhältnis 1 : 20, überimpft werden.

Bevorzugterweise zieht man die Vorkultur auf der gleichen Nährlösung an und läßt sie etwa 48 Stunden wachsen. Der Produzentenstamm scheidet die 2-(3'-Hydroxy-pyrid-2'-yl)-4-methyl-2-thiazolin-4-carbonsäure (eisenfrei) in die Kulturlösung aus.

Bei der erfindungsgemäßen Fermentation entstehen außer 2-(3'-Hydroxy-pyrid-2'-yl)-4-methyl-2-thiazolin-4-carbonsäure noch weitere Produkte, von denen $\beta$- und $\gamma$-Rubromycine identifiert werden konnten, die ursprünglich als Metabolite von Streptomyces collinus Lindenbein beschrieben worden waren [H. Brockmann und K.H. Renneberg, Naturwiss. 40, 59—60 (1953); H. Brockmann, W. Lenk, G. Schwanthe und A. Zeeck, Tetrahedron Letters 1966, 3225; H. Brockmann und A. Zeeck, Chem. Ber. 103, 1709—1726 (1970)].

Die Isolierung der 2-(3'-Hydroxy-pyrid-2'-yl)-4-methyl-2-thiazolin-4-carbonsäure kann nach an sich bekannten Methoden unter Berücksichtigung der chemischen, physikalischen und biologischen Eigenschaften des Antibiotikums erfolgen. Bevorzugterweise versetzt man die Kulturen bei einem pH-Wert in der Nähe des Neutralpunktes, z. B. bei pH 7,5, mit einer zur Komplexierung des Antibiotikums ausreichenden Menge eines Schwermetallsalzes, das mit dem Antibiotikum stabile isolierbare Komplexe bildet, z. B. mit Eisen(III)chlorid -Hexahydrat oder anderen Eisen(III)salzen.

Bei Zugabe von Eisen(III)salzen bildet sich ein charakteritisch gefärbter Eisenkomplex (»Ferrithiocin«), der sich bei neutralem pH und Kochsalzsättigung mit Essigester extrahieren läßt. Die Extraktion gelingt auch mit Methylenchlorid/iso-Propanol 85 : 15 bei pH 8,5 nach dem Extrelut®-Verfahren [J. Breiter, Kontakte Merck, 3, 9—14 (1977)], wobei Eisenionen aus dem Füllmaterial der Extraktionssäule zur Komplexbildung ausreichen. Die Reinigung der Extrakte erfolgt durch Extraktion der lipophilen Bestandteile mit Methylenchlorid aus Wasser und Chromatographieren an Sephadex LH-20. Der so gewonnene Eisenkomplex Ferrithiocin kann ohne weitere Reinigung aus Methanol/Acetonitril/Toluol kristallisiert werden.

Bei der milden Oxidation dieses Eisenkomplexes mit Kaliumpermanganat in wässriger Natronlauge entsteht ein Oxidationsprodukt mit einem Molekularionpik m/z = 192. Durch hochauflösendes Massenspektrum wird ihm die Summenformel $C_9H_8N_2OS$ zugeordnet. Im $^1H$-NMR-Spektrum des Oxidationsproduktes erscheint anstelle des AB Systems der ursprünglichen Methylengrupe ein olefinisches Singulett bei 6,94 ppm. Gleichzeitig wird das Singulett der tertiären Methylgruppe nach tieferem Feld auf 2,48 ppm verschoben.

Aufgrund dieser Daten handelt es sich bei dem Oxidationsprodukt um 3-Hydroxy-2-(4'-methyl-thiazol-2'-yl)-pyridin der Formel

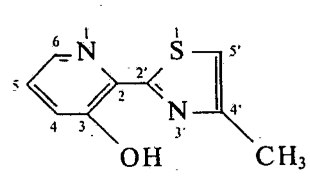

Die Behandlung des Eisenkomplexes Ferrithiocin mit $OH^{\ominus}$-Ionen in ausreichender Konzentration,

bevorzugterweise in Form von Alkalimetallhydroxid, z. B. mit 1 N NaOH, Abfiltrieren von Eisenhydroxyd und Ansäuern auf pH 3 liefert 2-(3'-Hydroxy-pyrid-2'yl)-4-methyl-2-thiazolin-4-carbonsäure in schwefelgelben, filzigen Nadeln. Das Massenspektrum zeigt einen Molekularionpik mit m/z 238, dem durch Hochauflösung die Summenformel $C_{10}H_{10}N_2O_3$ S zugeordnet wird. Die Mikroanalyse paßt am besten auf eine Verbindung mit einer Molekel Wasser, das aber beim scharfen Trocknen wieder verloren geht. Im IR-Absorptionsspektrum weist die breite Absorptionsbande zwischen 3600 und 2200 $cm^{-1}$ sowie eine Carbonylbande bei 1695 $cm^{-1}$ auf das Vorhandensein einer Carboxylgruppe hin. Im $^{13}C$ NMR Spektrum wird das Signal bei 180 ppm dem Carboxyl-Kohlenstoffatom zugeordnet. Das 3. Sauerstoffatom wird einer phenolischen, wasserstoffbrückengebundenen OH Gruppe zugeordnet. Im IR-Spektrum findet man die dazugehörende Bande bei 3550 $cm^{-1}$ und im $^1H$-NMR-Spektrum des entsprechenden 4-Carbonsäure-Methylesters und des oben angeführten Oxidationsproduktes 3-Hydroxy-2-(4'-methyl-thiazol-2'-yl)-pyridin ein mit $D_2O$ austauschbares Singulett bei 12 ppm. Eine tertiär gebundene Methylgruppe gibt im $^1H$-NMR-Spektrum ein Singulett bei 1,61 ppm und eine Methylengruppe ein AB-System bei 3,30 und 3.75 ppm J = 12 Hz. Im $^{13}C$-NMR-Spektrum findet man die entsprechenden Signale als Quartett bei 25,5 ppm und als Triplett bei 40,7 ppm. Die gemäß Bruttoformel noch verbleibenden 3 Wasserstoffatome sind an einen Aromaten gebunden (Signale bei 7,45 ppm, 2 H, und 8,18 ppm, 1H, im $^1H$-NMR; Dublette bei 126,0, 128,1 und 141,1 ppm im $^{13}C$-NMR-Spektrum).

Über die Art und das Substitutionsmuster des aromatischen Rings der durch Behandlung des Ferrithiocins mit Natronlauge erhaltenen eisenfreien Verbindung gibt eine saure Hydrolyse Auskunft. Nach Behandlung mit 6 N HCl bei 90° während 1 Stunde isoliert man mittels präparativer DC eine aromatische Carbonsäure ($C_6H_5NO_3$, $M^+$ 139), aus der man durch Veresterung mit Diazomethan eine Verbindung erhält, die als 3-Hydroxy-picolinsäuremethylester identifiziert wird. Die Struktur ergibt sich aus dem Massenspektrum: $M^+$ = 253, m/z 223 ($M^+$ $-CH_2$ = O, Mac Lafferty Umlagerung), 221 ($M^+$ $-CH_3OH$), 95 ($M^+$ $-CH_2$ = CO) usw., sowie aus dem $^1$NMR- und IR-Spektrum, die mit den Spektren einer authentischen Probe dieser Verbindung übereinstimmen.

Eine weitere Komponente der sauren Hydrolyse, die auf Dünnschicht (DC)-Platten mit Nitroprussid-Natrium [vgl. G. Tonnies und J.J. Kolb, Anal. Chemistry 23, 823 (1951)] anfärbbar ist, läßt sich mittels Chromatographie an CM Sephadex C-25 in PyridinyAcetatpuffer pH 5 isolieren.

Diese Verbindung erweist sich nur bei tiefen Temperaturen (−20° C) und unter Stickstoff als stabil. Ein stabileres, gemäß DC und $^1$NMR-Spektrum nicht ganz einheitliches Derivat der Formel

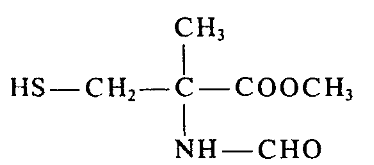

erhält man durch Behandlung des Rohproduktes mit Diazomethan in absolutem Dimethylformamid unter Zugabe von $BF_3$-Etherkomplex und anschließender Reinigung auf präparativer DC-Platte. Im $^1H$-NMR-Spektrum findet man ein Methylestersignal bei 3,78 ppm sowie bei 1,67 ppm das Singulett einer tertiären Methylgruppe. Bei 1,35 ppm erscheint ein Doppeldublett entsprechend 1 H mit einer Kopplungskonstanten J von 8 und 10 Hz, das bei Zugabe von $D_2O$ verschwindet. Dieses Signal wird einer primären SH-Gruppe zugeordnet. Der Kopplungspartner, eine vicinale $CH_2$-Gruppe, ergibt sich durch 2 Doppeldubletten bei 2,96 ppm mit J von 10 bis 14 Hz sowie bei 3,55 mit J von 8 und 14 Hz zu erkennen. Diese Signalgruppe wird bei $D_2O$-Zugabe zu 2 Dubletten mit J von 14 Hz reduziert. Eine N-Formylgruppe erkennt man aufgrund eines Dubletts mit J von 2 Hz bei 8,17 ppm sowie des breiten Signals eines Amidprotons bei 6,6 ppm. Offentlichtlich ist unter den gewählten Reaktionsbedingungen die Formylgruppe vom Lösungsmittel DMF auf den Stickstoff des Abbauproduktes übertragen worden. Anhand dieser Signale ergibt sich für dieses Abbauprodukt die o. a. Struktur (N-Formyl-2-methylcysteinmethylester). Das Massenspektrum zeigt zwar keinen Molekularpik ($C_6H_{11}NO_3S$, M.G. 177), die beobachteten Fragmente passen aber gut auf die Struktur m/z 132 ($M^+$ −HN = CH−OH, Mac Lafferty Umlagerung, 100% Int.), 118 ($M^+$ $-COOCH_3$), 100 ($M^+$ −NH = CH−OH und $CH_3OH$). Dieses Abbauergebnis weist auf ein Thiazolinderivat hin.

Verbindungen der Formel (I), in denen die 4-Carboxy-Gruppe verestert (vgl. u.) oder z. B. durch milde Oxidation mit Kaliumpermanganat entfernt ist, zeigen mit $FeCl_3$-Lösung wohl noch eine Farbreaktion, bilden aber keine isolierbaren Komplexe mehr. Dies weist darauf hin, daß die freie 4-Carboxy-Gruppe zur Komplexbildung essentiell ist.

Die erfindungsgemäß erhältlichen Verbindungen der Formel (I) können in an sich bekannter Weise in andere Verbindungen der Formel (I) überführt werden.

In den Verbindungen der Formel (I), in denen $R^1$ eine freie Hydroxylgruppe bedeutet, kann diese nach an sich bekannten Methoden verethert oder verestert werden. Eine freie 4-Carboxy-Gruppe kann verestert werden.

Geeignete Mittel zur Veretherung der 3'-OH-Gruppe und zur Veresterung der Carboxylgruppe sind beispielsweise entsprechende Diazoverbindungen, wie gegebenenfalls substituierte Diazoniederalkane, z. B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z. B. Methylenchlorid, oder eines Ethers, wie eines Diniederalkylethers, z. B. Diethylether, oder eines cyclischen Ethers, z. B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäß und/oder einer Inertgas-, z. B. Stickstoffatomosphäre, zur Anwendung gebracht.

Behandelt man beispielsweise 2-(3'-Hydroxy-pyrid-2'-yl)-4-methylthiazolin-4-carbonsäure bei 0° mit Diazomethan, erhält man den 4-Carbonsäuremethylester, der im IR-Spektrum ($CHCl_3$) bei 1735 $cm^{-1}$ die Bande eines aliphatischen Esters und im $^1H$-NMR-Spektrum ein zusätzliches Singulett entsprechend 3 H bei 3,77 ppm zeigt. Führt man die gleiche Reaktion erschöpfend bei Raumtemperatur aus, wird auch die phenolische 3'-Hydroxygruppe methyliert, was aus dem Verschwinden des Signals bei 12 ppm und dem Entstehen eines Singuletts bei 3,88 ppm im $^1H$-NMR hervorgeht.

Weitere geeignete Mittel zur Veretherung der 3'-OH-Gruppe und zur Veresterung der Carboxylgruppe sind Ester entsprechender Alkohole, in erster Linie solche mit starken anorganischen oder organischen Säuren, wie Mineralsäuren, z. B. Halogenwasserstoffsäuren, wie Chlorwasserstoff-, Bromwasserstoff- oder Jodwasserstoffsäure, ferner Schwefelsäure, oder Halogen-schwefelsäure, z. B. Fluorschwefelsäure, oder starken organischen Sulfonsäuren, wie gegebenenfalls, z. B. durch Halogen, wie Fluor, substituierten Niederalkansulfonsäuren, oder aromatischen Sulfonsäuren, wie z. B. gegebenenfalls durch Niederalkyl, wie Methyl, Halogen, wie Brom, und/oder Nitro substituierten Benzolsulfonsäuren, z. B. Methansulfon-, Trifluormethansulfon- oder p-Toluolsulfonsäure. Solche Ester sind u. a. Niederalkylhalogenide, Diniederalkylsulfate, wie Dimethylsulfat, ferner Fluorsulfonsäureester, wie -niederalkylester, z. B. Fluorsulfonsäuremethylester, oder gegebenenfalls Halogen-substituierte Methansulfonsäure-niederalkylester, z. B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z. B. Methylenchlorid, eines Ethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z. B. Natrium- oder Kaliumcarbonat oder -hydrogencarbonat (üblicherweise zusammen mit einem Sulfat), oder organische Basen, wie üblicherweise sterisch gehinderte Triniederalkylamine, z. B. N,N-Diisopropyl-N-ethyl-amin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z. B. bei Temperaturen von etwa —20° C bis etwa 50° C und, wenn notwendig, in einem geschlossenen Gefäß und/oder in einer Inertgas-, z. B. Stickstoffatmosphäre, gearbeitet wird.

Durch Phasentransfer-Katalyse [siehe Dehmlow, Angewandte Chemie, Bd. 5, S. 187 (1974)] kann die oben beschriebene Veretherungsreaktion wesentlich beschleunigt werden. Als Phasentranfer-Katalysatoren können quartäre Phosphoniumsalze und insbesonder quartäre Ammoniumsalze, wie gegebenenfalls substituierte Tetraalkylammoniumhalogenide, z. B. Tetrabutylammoniumchlorid, -bromid oder -jodid, oder auch Benzyl-triethylammoniumchlorid, in katalytischer oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten, niederaliphatischen, cycloaliphatischen oder aromatischen Kohlenwasserstoffe, wie Tri- oder Tetrachlorethylen, Tetrachlorethan, Tetrachlorkohlenstoff, Chlorbenzol, Toluol oder Xylol. Die als Kondensationsmittel geeigneten Alkalimetallcarbonate oder -hydrogencarbonate, z. B. Kalium- oder Natriumcarbonat oder -hydrogencarbonat, Alkalimetallphosphate, z. B. Kaliumphosphat, und Alkalimetallhydroxide, z. B. Natriumhydroxid, können bei basenempfindlichen Verbindungen der Reaktionsmischung titrimetrisch, z. B. mittels eines Titrierautomaten, zugesetzt werden, damit der pH-Wert während der Veretherung zwischen etwa 7 und etwa 8,5 bleibt.

Weitere Mittel zur Veretherung der 3'-OH-Gruppe oder zur Veresterung der 4-Carboxylgruppe sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z. B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorant imonat. Man verwendet diese Veretherungsmittel vorzugsweise in einem inerten Lösungsmittel, wie einem Ether oder einem halogenierten Kohlenwasserstoff, z. B. Diethylether, Tetrahydrofuran oder Methylenchlorid, oder in einem Gemisch davon, wenn notwendig, in Gegenwart einer Base, wie einer organischen Base, z. B. eines, vorzugsweise sterisch gehinderten, Triniederalkylamins, z. B. N,N-Diisopropyl-N-ethyl-amin, und unter Kühlen, bei Raumtemperatur oder unter leichtem Erwärmen, z. B. bei etwa —20° C bis etwa 50° C, wenn notwendig, in einem geschlossenen Gefäß und/oder in einer Inertgas-, z. B. Stickstoffatmosphäre.

Weitere Veretherungsmittel sind schließlich entsprechende 1-substituierte 3-Aryltriazenverbindungen, worin der Substituent den verethernden Rest, und Aryl vorzugsweise gegebenenfalls substituiertes Phenyl, z. B. Niederalkylphenyl, wie 4-Methylphenyl, bedeutet. Solche Triazenverbindungen sind 3-Aryl-1-niederalkyltriazene, z. B. 3-(4-Methylphenyl)-1-methyl-triazen, 3-(4-Methylphenyl)-1-ethyltriazen oder 3-(4-Methylphenyl)-1-isopropyl-triazen. Diese Reagentien werden üblicherweise in Gegenwart von inerten Lösungsmitteln, wie gegebenenfalls halogenierten Kohlenwasserstoffen oder Ethern, z. B. Benzol, oder Lösungsmittelgemischen, und unter Kühlen, bei Raumtemperatur und vorzugsweise bei erhöhter Temperatur, z. B. bei etwa 20°C bis etwa 100°C, wenn notwendig in einem geschlossenen Gefäß und/oder in einer Inertgas-, z. B. Stickstoffatmosphäre, verwendet.

Die Umwandlung von freiem Carboxyl in einer Verbindung der Formel (I) in verestertes Carboxyl, kann weiterhin beispielsweise erfolgen, indem man eine Verbindung der Formel (I), worin andere, gegebenenfalls vorhandene funktionelle Gruppen gegebenenfalls in geschützter Form vorliegen, oder ein reaktionsfähiges funktionelles Carboxyderivat, inkl. ein Salz davon, mit einem entsprechenden Alkohol oder einem reaktionsfähigen funktionellen Derivat davon umsetzt.

Die Veresterung von freiem Carboxyl mit dem gewünschten Alkohol wird in Gegenwart eines geeigneten Kondensationsmittels durchgeführt. Übliche Kondensationsmittel sind z. B. Carbodiimide, beispielsweise N,N'-Diethyl-, N,N'-Dipropyl, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z. B. 2-Ethyl-5-phenyl-1,2-oxazolium-3'-sulfonat und 2-tert.-Butyl-5-methyl-isoxazolium-perchlorat, oder eine geeignete Acylaminoverbindung, z. B. 2-Ethoxy-1-ethoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z. B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel (I) sind z. B. Anhydride, insbesondere gemischte Anhydride, und aktivierte Ester.

Gemischte Anhydride sind z. B. diejenigen mit anorganischen Säuren, wie Halogenwasserstoffsäuren, d. h. die entsprechenden Säurehalogenide, z. B. -chloride oder -bromide, ferner Stickstoffwasserstoffsäuren, d. h. die entsprechenden Säureazide, sowie phosphorhaltigen Säuren, z. B. Phosphorsäure, Diethylphosphorsäure oder phosphorige Säure, oder schwefelhaltigen Säuren, z. B. Schwefelsäure, oder Cyanwasserstoffsäure. Gemischte Anhydride sind weiter z. B. diejenigen mit organischen Carbonsäuren, wie mit gegebenenfalls, z. B. durch Halogen, wie Fluor oder Chlor, substituierten Niederalkancarbonsäuren, z. B. Pivalinsäure oder Trichloressigsäure, oder mit Halbestern, insbesondere Niederalkylhalbestern der Kohlensäure, wie Ethyl- oder Isobutylhalbestern der Kohlensäure, oder mit organischen, insbesondere aliphatischen oder aromatischen Sulfonsäuren, z. B. p-Toluolsulfonsäure.

Zur Reaktion mit dem Alkohol geeignete aktivierte Ester sind z. B. Ester mit vinylogen Alkoholen (d. h. Enolen), wie vinylogen Niederalkenolen, oder Iminomethylesterhalogeniden, wie Dimethyliminomethylesterchlorid (hergestellt aus der Carbonsäure und Dimethylchlormethyliden-iminium-chlorid der Formel

$$[(CH_3)_2N = CHCl]^{\oplus}Cl^{\ominus})$$

oder Arylester, wie Pentachlorphenyl-, 4-Nitrophenyl- oder 2,3-Dinitrophenylester, heteroaromatische Ester, wie Benztriazol-, z. B. 1-Benztriazolester, oder Diacyliminoester, wie Succinylimino- oder Phthalyliminoester.

Die Acylierung mit einem solchen Säurederiat, wie einem Anhydrid, insbesondere mit einem Säurehalogenid, wird bevorzugt in Anwesenheit eines säurebindenden Mittels, beispielsweise einer organischen Base, wie eines organischen Amins, z. B. eines tertiären Amins, wie Triniederalkylamin, z. B. Trimethylamin, Triethylamin oder Ethyldiisopropylamin, oder N,N-Diniederalkyl-anilin, z. B. N,N-Dimethyl-anilin, oder eines cyclischen tertiären Amins, wie eines N-niederalkylierten Morpholins, wie N-Methylmorpholin, oder einer Base vom Pyridin-Typ, z. B. Pyridin, einer anorganischen Base, beispielsweise eines Alkalimetall- oder Erdalkalimetallhydroxids, -carbonats oder -hydrogencarbonats, z. B. Natrium-, Kalium- oder Calciumhydroxid, -carbonat oder -hydrogencarbonat, oder eines Oxirans, beispielsweise eines niederen 1,2-Alkylenoxids, wie Ethylenoxid oder Propylenoxid, durchgeführt.

Ein reaktionsfähiges funktionelles Derivat des veresternden Alkohols ist in erster Linie ein entsprechender Ester, vorzugsweise mit einer starken anorganischen oder organischen Säure und stellt insbesondere ein entsprechendes Halogenid, z. B. Chlorid, Bromid oder Jodid, oder eine entsprechende Niederalkyl- oder Aryl-, wie Methyl- oder 4-Methylphenylsulfonyloxyverbindung dar.

Ein solcher reaktionsfähiger Ester eines Alkohols kann mit der freien Carboxylverbindung der Formel (I) oder einem Salz, wie einem Alkalimetall- oder Ammoniumsalz, davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z. B. Dimethylformamid, einem hylogenierten Kohlenwasserstoff, z. B. Methylenchlorid, Tetrachlorkohlenstoff oder Chlorbenzol, einem Keton, z. B. Aceton, einem Ester, z. B. Essigsäureethylester, oder einem Nitril, z. B. Acetonitril, oder Mischungen davon, wenn notwendig unter Kühlen oder Erwärmen, z. B. in einem Temperaturbereich von etwa —40° C bis +100° C, vorzugsweise bei etwa —10° C bis etwa +40° C, und/oder in einer Inertgas-, z. B. Stickstoffatmosphäre.

Ferner kann das Säurederivat, wenn erwünscht, in situ gebildet werden. So erhält man z. B. ein gemischtes Anhydrid durch Behandeln der Carbonsäureverbindung mit entsprechend geschützten funktionellen Gruppen oder eines geeigneten Salzes davon, wie eines Ammoniumsalzes, z. B. mit einem organischen Amin, wie Piperidin oder 4-Methylmorpholin, oder eines Metall-, z. B. Alkalimetallsalzes mit einem geeigneten Säurederivat, wie dem entsprechenden Säurehalogenid einer gegebenenfalls substituierten Niederalkancarbonsäure, z. B. Trichloracetylchlorid, mit einem Halbester eines Kohlensäure-halbhalogenids, z. B. Chlorameisensäurehalbester oder -isobutylester, oder mit einem Hylogenid einer Diniederalkylphosphorsäure, z. B. Diethylphosphorbromidat, und verwendet das so erhältliche gemischte Anhydrid ohne Isolierung.

Zur Veresterung kann man die Hydroxygruppe durch Behandeln des Ausgangsmaterials der Formel (I) mit einem, den gewünschten Acylrest einer organischen Carbonsäure einführenden Acylierungsmittel in eine Acyloxygruppe umwandeln. Dabei verwendet man die entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z. B. diejenigen mit Halogenwasserstoffsäuren, d. h. die entsprechenden Säurehalogenide, insbesondere -chloride, ferner mit Cyanwasserstoffsäure, oder dann diejenigen mit geeigneten Kohlensäurehalbderivaten, wie entsprechenden -halbestern (wie die z. B. mit einem Halogen-ameisensäure-niederalkyl, wie Chlorameisensäure-ethylester oder -isobutylester, gebildeten gemischten Anhydride) oder mit gegebenenfalls substituierten, z. B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z. B. diejenigen von organischen Carbonsäuren, d. h. Ketene, wie Keten oder Diketen, oder diejenigen von Carbamin- oder Thiocarbaminsäuren, d. h. Isocyanate oder Isothiocyanate. Weitere reaktionsfähige, als Acylierungsmittel verwendbare Derivate von organischen Carbonsäuren sind aktivierte Ester, wie geeignet substituierte Niederalkyl-, z. B. Cyanmethylester, oder geeignet substituierte Phenyl-, z. B. Pentachlorphenyl- oder 4-Nitrophenylester. Die Veresterung kann, wenn notwendig, in Gegenwart von geeigneten Kondensationsmitteln, bei Verwendung von freien Carbonsäuren, z. B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z. B. in Gegenwart von basischen Mitteln, wie Triniederalkylaminen, z. B. Triethylamin, oder heterocyclischen Basen, z. B. Pyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäß und/oder in einer Inertgas-, z. B. Stickstoffatmosphäre, durchgeführt werden. Geeignete Lösungsmittel sind z. B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Eine durch eine organische Sulfonsäure, z. B. Niederalkansulfonsäure, wie Methansulfonsäure, oder eine aromatische Sulfonsäure, wie p-Toluolsulfonsäure, veresterte Hydroxygruppe kann man vorzugsweise durch Behandeln des Ausgangsmaterials der Formel (I) mit einem reaktionsfähigen Sulfonsäurederivat, wie einem entsprechenden Halogenid, z. B. Chlorid, wenn notwendig, in Gegenwart eines Säure-neutralisierenden basischen Mittels, z. B. einer anorganischen oder organischen Base, z. B. in analoger Weise wie die entsprechenden Ester mit organischen Carbonsäuren, bilden.

In einer erhaltenen Verbindung der Formel (I), worin eine oder mehrere funktionelle Gruppen geschützt sind, können diese, z. B. geschützte Carboxyl-, und/oder Hydroxygruppen, in an sich bekannter Weise, mittels Solvolyse, insbesondere Hydrolyse, Alkoholyse oder Acidolyse, oder in einigen Fällen auch mittels vorsichtiger Reduktion, gegebenenfalls stufenweise oder gleichzeitig freigesetzt werden. Silylschutzgruppen werden vorteilhafterweise mit Fluoriden, z. B. Tetraethylammoniumfluorid, abgespalten.

Salze von Verbindungen der Formel (I) mit salzbildenden Gruppen können in an sich bekannter Weise hergestellt werden. So kann man Salze von Verbindungen der Formel (I) mit sauren Gruppen, z. B. durch Behandeln mit Metallverbindungen, wie Alkalimetallsalzen von geeigneten organischen Carbonsäuren, z. B. dem Natriumsalz der $\alpha$-Ethyl-capronsäure, oder mit anorganischen Alkali- oder Erdalkalimetallsalzen, z. B. Natriumhydrogencarbonat, oder mit Ammoniak oder einem geeigneten organischen Amin bilden, wobei man vorzugsweise stöchiometrische Mengen oder nur einen kleinen Überschuß des salzbildenden Mittels verwendet. Säureadditionssalze von Verbindungen der Formel (I) erhält man in üblicher Weise, z. B. durch Behandeln mit einer Säure oder einem geeigneten Anionenaustauschreagens. Innere Salze von Verbindungen der Formel (I), welche z. B. eine freie Carboxylgruppe enthalten, können z. B. durch Neutralisieren von Salzen, wie Säureadditionssalzen, auf den isoelektrischen Punkt, z. B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z. B. durch Behandeln mit geeigneten Säuren, und Säureadditionssalze z. B. durch Behandeln mit einem geeigneten basischen Mittel.

Die Eisen-, Aluminium- oder Chromkomplexe der Verbindungen der Formel (I) mit freier 4-Carboxy-Gruppe werden z. B. hergestellt, indem man ein Salz des entsprechenden Metalls, z. B. ein Halogenid, wie Chlorid, mit einer Verbindung der Formel (I), worin der Rest $COOR^2$ eine freie Carboxylgruppe bedeutet, oder mit einem geeigneten Salz dieser Säure, z. B. einem Alkalimetallsalz, wie Natrium- oder Kaliumsalz, vorteilhafterweise in einem polaren Lösungsmittel oder -gemisch, z. B. in wässeriger oder alkoholischer, z. B. methanolischer, Lösung, die einen geeigneten pH-Wert hat, zusammenbringt. Die Reaktionstemperatur liegt normalerweise zwischen Schmelz- und Siedepunkt bei Normaldruck des verwendeten Lösungsmittels oder Lösungsmittelgemisches, in der Regel zwischen 15° und 50° C, z. B. zwischen 20° und 40° C, z. B. bei 37°.

Da die erfindungsgemäßen Komplexverbindungen sowohl unter stark sauren, z. B. bei pH = 1, als auch unter stark basischen, z. B. bei pH = 12, Bedingungen instabil sind, wird der pH-Wert der Reaktionslösung vorteilhafterweise zwischen 3 und 10, insbesondere zwischen 5 und 8, z. B. in Höhe eines physiologischen pH-Werts, wie 7,5, gehalten. Die genauen Grenzen des pH-Bereichs, bei dem die Komplexbildung möglich ist, hängen vor allem von der Art der Komplexbildungspartner, z. B. von der Art des Metallions und der Löslichkeit von dessen Hydroxid(en) ab. Unter den genannten optimalen Bedingungen läuft die Komplexbildung sehr rasch ab, so daß sich das Gleichgewicht zwischen den Komplex und den Ausgangsstoffen innerhalb von wenigen Minuten oder Stunden einstellt.

Gemische von Isomeren können in an sich bekannter Weise, z. B. durch fraktionierte Kristalliation, Chromatographie etc. in die einzelnen Isomeren aufgetrennt werden.

Racemate können in sich bekannter Weise, z. B. nach Überführung der optischen Antipoden in Diastereomere, beispielsweise durch Umsetzung mit optisch aktiven Säuren oder Basen, gespalten werden.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemäßen Verfahren erhältliche Verbindung unter den Verfahrensbedingungen erzeugt und in situ weiterverarbeitet.

Die Ausgangsstoffe sind im Handel erhältlich und/oder bekannt oder können nach bekannten Verfahren hergestellt werden.

Vorzugsweise werden solche Ausgangsstoffe verwendet und die Reaktionsbedingungen so gewählt, daß man zu den in dieser Anmeldung als besonders bevorzugt aufgeführten Verbindungen gelangt.

Die pharmazeutisch verwendbaren Verbindungen der vorliegenden Erfindung können z. B. zur Herstellung von pharmazeutischen Präparaten verwendet werden, welche eine wirksame Menge der Aktivstubstanz zusammen oder im Gemisch mit anorganischen oder organischen, festen oder flüssigen, pharmazeutisch verwendbaren Trägerstoffen enthalten.

Bei den erfindungsgemäßen pharmazeutischen Präparaten handelt es sich um solche zur enteralen, wie oralen, sowie parenteralen, wie subcutanen, Verabreichung an Warmblüter, welche den pharmakologischen Wirkstoff allein oder zusammen mit einem pharmazeutisch anwendbaren Trägermaterial enthalten. Die Dosierung des Wirkstoffes hängt von der Warmblüter-Spezies, dem Alter und dem individuellen Zustand, der zu behandelnden Krankheit sowie von der Applikationsweise ab.

Die neuen pharmazeutischen Präparate enthalten von etwa 10% bis etwa 95%, vorzugsweise von etwa 20% bis etwa 90% des Wirkstoffes. Erfindungsgemäße pharmazeutische Präparate können z. B. in Dosiseinheitsform, wie Dragées, Tabletten, Kapseln, Suppositorien oder Ampullen, vorliegen.

Die pharmazeutischen Präparate der vorliegenden Erfindung werden in an sich bekannter Weise, z. B. mittels konventioneller Misch-, Granulier-, Dragier-, Lösungs- oder Lyophilisierungsverfahren, hergestellt. Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabeletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyyrolidon, Agar, Alginsäure oder ein Salz davon, wie Natriumalginat. Hilfsmittel sind in erster Linie Fließregulier- und Schmiermittel, z. B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Überzügen versehen, wobei man u. a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen

oder, zur Herstellung von magensaftresistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z. B. zur Indentifizierung oder zur Kennzeichnung verschiedener Wirkstoffdosen, beigefügt werden.

Die folgenden Beispiele dienen zur Illustration der Erfindung. Temperaturen werden in Celsiusgraden angegeben.

Algemeines: Smp. in offener Kapillare bestimmt, nicht korrigiert.

UV: Perkin-Elmer-Spektrometer 402,

Angaben von $\lambda_{max}$ in nm (log$\varepsilon$).

IR: Perkin-Elmer-Spektrometer 157 : G; Angaben in $cm^{-1}$.

[1]HMR: Varian-Spektrometer HA-100 (100 MHz); chemische Verschiebungen in $\delta$ [ppm] gegenüber Tetramethylsilan (TMS).

$^{13}$C-NMR: Varian-Spektrometer XL-100 (25,3 MHz); chemische Verschiebungen in $\delta$ [ppm] gegenüber TSM (in Klammern Aufspaltung im offresonance-Spektrum).

MS: Intensität in % des Basispeaks.

DC: Kieselgel-60-Fertigplatten $F_{254}$ Merck; die Lokalisierung der Flecken erfolgte im allgemeinen durch Betrachten im UV-Licht oder Entwicklung im Joddampf.

## Beispiel 1

Der Stamm Streptomyces antibioticus Tü 1998 wird im 10 l Fermenter (New Brunswick, Modell F 14) 90 Stunden bei 27°, 4 l Luft/Min. und 220 U/Min. auf der folgenden Nährlösung angezogen: 2% vollfettes Sojamehl und 2% Mannit in Wasser, pH der Nährlösung vor dem Sterilisieren mit Natronlauge auf pH 7,5 gebracht. Als Impfmaterial dienen 5% einer 48 Stunden alten Vorkultur, die auf der gleichen Nährlösung angezogen wird. Zur Aufarbeitung werden die Kulturen bei unverändertem pH mit 12 g Eisen-(III)-chlorid Hexahydrat versetzt und unter Zusatz von 1,5% Celit filtriert. Das Filtrat wird mit Natronlauge auf pH 7 eingestellt, mit Kochsalz gesättigt und 4mal mit 2,5 l Essigester extrahiert. Die Extrakte engt man im Vakuum zum braunen Sirup ein, löst diesen in 50 ml Wasser und extrahiert zur Entfernung lipophiler Bestandteile 4mal mit Methylenchlorid. Die wässerige Phase wird im Vakuum zum trockenen Pulver eingeengt, dieses in 10 ml Chloroform/Methanol 1 : 1 gelöst und an Sephadex LH 20 chromatographiert. Die gemäß DC einheitlichen Fraktionen ergeben den Eisenkomplex von 2-(3'Hydroxy-pyrid-2'-yl)-4-methyl-2-thiazolin-4-caroxylat (Ferrithiocin) als dunkelbraun-rotes Pulver.

Zur Analyse wird das amorphe Pulver aus Methanol/Acetonitril/Toluol zu kleinen, verfilzten Nadeln kristallisiert. Smp. 160° (Zers.). DC: Rf 0,27 in Chloroform/Methanol/Wasser 65 : 25 : 4. $[\alpha]_D$ = 578 (c = 6,4.10$^{-3}$ in $H_2O$. UV (c = 1% $H_2O$) 207 (2,82), 227 Sch. (2,71), 268 Sch. (2,4), 325 (2,48), 405 (2,04), 460 Sch. (1,9).

47,5 mg des erhaltenen Ferrithiocins löst man in 2 ml $H_2O$ stellt mit 1 N NaOH auf pH 10 ein und tropft eine gesättigte, wässerige $KMnO_4$-Lösung so langsam dazu, daß stets nur eine schwache Violettfärbung vorhanden ist. Sobald das Ausgangsprodukt vollständig (DC) umgesetzt ist, filtriert man vom Braunstein und Eisenhydroxid ab und extrahiert 2mal mit 10 ml $CHCl_3$. Die Extrakte liefern nach dem Einengen im Vakuum weißes rohres 3-Hydroxy-2-(4'-methyl-thiazol-2'-yl)-pyridin, das aus Aceton/Wasser zu farblosen Nadeln kristallisiert.

DC: Rf 0,41 in $CHCl_3$ · Smp. 115°.

IR ($CHCl_3$): 1580, 1525, 1450, 1025.

$^1$H-NMR ($CDCl_3$): $\varepsilon$ = 2,48 (s; 3H), 6,94 (s; 1H); 7,1—7,46 (m; 2H), 8,15 (dd, $J_1$ = 2Hz), $J_2$ = 4H; 1H), 12,02 (br. s. —OH).

MS: u. a. 194 (6), 193 (14), 192,0357 ($M^+$, ber. für $C_9H_8N_2OS$: 192,0357,100), 194 (22), 72 (33), 57 (26).

## Beispiel 2

Zu 97 mg Ferrithiocin werden 3 ml 1 N NaOH gegeben, mit Hilfe von Celit vom ausgeschiedenen Eisenhydroxid abfiltriert und das Filtrat bei 0° mit konz. Salzsäure auf pH 3 eingestellt. Dabei kristallisiert 2-(3'-Hydroxy-pyrid-2'-yl)-4-methyl-2-thiazolin-4-carbonsäure in schwefelgelben Nadeln aus, die mit eiskaltem Wasser gewaschen werden. Eine Analysenprobe wird aus heißem Wasser/Methanol umkristallisiert.

DC: Rf 0,17 in $CHCl_3$/Methanol/Wasser 65 : 25 : 4, Smp. 90—92°, $\alpha_D$ = +30,1° (c = 1,01, Methanol).

UV ($H_2O$): 200 (4,18), 235 Sch. (3,73), 308 (3,79), 382 (3,33).

$^1$H-NMR (100 MHz, $d_6$-DMSO): $\delta$ = 1,61 (s; 3H), 3,30 (d,J = 12Hz, 1H), 3,75 (d, J = 12Hz, 1H), 7,45 d, J = 3Hz; 2H), 8,18 (t, J = 3Hz; 1H), 12—14 (br, 2H).

$^{13}$C-NMR (25 MHz, $CD_3OD$): $\delta$ = 25,5 (q), 40,7 (t), 87,3 (s), 126,0 (d), 128,1 (d), 135,3 (s), 141,1 (d), 156,7(s), 172,8 (s), 180,0 (s).

MS: u. a. 240 ($M^+$ + 2,3), 238,0404 ($M^+$ ber. für $C_{10}H_{10}N_{203}S$: 238,0412, 26), 193 (82), 121 (19), 92 (86), 91 (100), 65 (27), 39 (26).

$C_{10}H_{10}N_2O_3S \cdot H_2O$ (256,05)

| | | | | |
|---|---|---|---|---|
| Ber. | C 46,86 | H 4,72 | N 10,93 | S 12,53 |
| Gef. | C 46,58 | H 4,77 | N 10,74 | S 12,51 |

Aus 124 mg Ferrithiocin wird wie oben beschrieben 2-(3'-Hydroxy-pyrid-2'-yl)-4-methyl-2-thiazolin-4-carbonsäure hergestellt und diese während 1 Stunde bei 90° in 2 ml 6N Salzsäure unter Stickstoffatmosphäre hydrolisiert. Der Eindampfrückstand enthält gemäß DC in Chloroform/Methanol/Wasser 65 : 25 : 4 2 Hauptprodukte mit Rf 0,17 (Joddampf und Nitroprussid — Na positiv, charakteristisch für —SH Verbindungen [s. Tonnies und J.J. Kolb, Anal. Chemistry 23, 823 (1951)] und Rf 0,32 [Fluoreszenzlöschung]. Das letztere kann durch präp. DC im gleichen Laufmittelsystem rein dargestellt werden: 3-Hydroxy-picolinsäure in Form eines farblosen Pulvers mit Smp. 219—223° (Zers.).

IR (KBr): 3430 br., 1635, 1605, 1455.

$^1$H-NMR ($CD_3OD$): 7,9 (br., 2H), 8,18 (br., 1H).

$^{13}$C-NMR ($CD_3OD$): 129,0 (d), 130,7 (d), 134,0 (s), 139,0 (d), 160,1 (s), 173,0 (s).

MS.: u. a. 139 ($M^+$, $C_6H_5NO_3$, 9), 121 (7), 95 (100), 93 (15), 67 (19), 44 (54).

Die Verbindung wird in 2 ml absolutem Dimethylformamid gelöst und unter Zusatz von 2 Tropfen $BF_3$-Etherkomplex mit etherischer Diazomethanlösung methyliert. Dann wird mit 10 ml $H_2O$ verdünnt und 2mal mit Essigester extrahiert. Die Extrakte werden mit $H_2O$ gewaschen, über $Na_2SO_4$ getrocknet und im Vakuum eingeengt. Der Rückstand kristallisiert aus Essigester/Hexan in farblosen Nadeln mit Smp. 72—73°. Die Verbindung erweist sich im direkten Vergleich (Mischsmp., DC, IR und $^1$H-NMR) als identisch mit einer authentischen Probe von 3-Hydroxy-picolinsäuremethylester.

$^1$H-NMR ($CDCl_3$): 4,02 (s, 3H), 7,3—7,45 (m, 2H), 8,18—8,32 (m, 1H), 10,58 (s, 1H).

MS: u. a. 153 ($M^+$, $C_7H_7NO_3$, 84) 123 (73), 95 (95), 93 (100).

40 mg (0,168 mMol)-2-(3'-Hydroxy-pyrid-2'yl)-4-methyl-2-thiazolin-4-carbonsäure werden wie oben beschrieben hydrolisiert und der Eindampfrückstand in Pufferlösung pH 5 (Pyridin/Eisessig 65 : 35 v : v je 0,1 M) an CM Sephadex C25 chromatographiert. Die einheitlichen mit Nitroprussid-Na anfärbbaren Fraktionen werden im Vakuum eingeengt und in absolutem Dimethylformamid unter Zusatz von $BF_3$-Etherkomplex mit etherischer Diazomethanlösung methyliert. Das Rohprodukt wird mit Essigester ausgeschüttelt, die Extrakte mehrfach mit $H_2O$ gewaschen, über $Na_2SO_4$ getrocknet und im Vakuum eingedampft, worauf man N-Formyl-2-methyl-cysteinmethylester als farbloses Öl erhält.

DC: Rf 0,41 in $CHCl_3$/Essigester 1 : 1 (mit Spuren von Verunreinigungen).

IR ($CHCl_3$): 3390, 2850, 1735, 1690.

$^1$H-NMR ($CDCl_3$): $\delta$ = 1,35 (dd, $J_1$ = 8 Hz, $J_2$ = 10 Hz; SH), 1,67 (s; 3H), 2,96 (dd, $J_1$ = 10 Hz, $J_2$ = 14 Hz; 1H), 3,55 (dd, $J_1$ = 8 Hz, $J_2$ = 14 Hz; 1H), 3,78 (s; 3H), 6,6 (br., 1H), 8,17 (d, J = 1 Hz, 1H).

Bei Zugabe von $D_2O$ verschwinden die Signale bei 1,35 und 6,6 ppm, die Doppeldubletten werden zu Dubletten mit J = 14 Hz und das Dublett bei 8,17 ppm zum Singulett.

MS: u. a. 178 ([$M^+$ + 1]$^+$, $C_6H_{12}NO_3S$, 1), 146 (3), 132 (100), 118 (21), 102 (29), 100 (62), 73 (22), 42 (54).

## Beispiel 3

70 mg (0,294 mMol) 2-(3'-Hydroxy-pyrid-2'-yl)-4-methyl-2-thiazolin-4-carbonsäure versetzt man während 20 Min. bei 0° in Methanol/Wasser mit etherischer Diazomethanlösung. Eindampfen im Vakuum, Ausschütteln mit $CHCl_3$ aus $H_2O$ und präp. DC (Kieselgel, Chloroform/Methanol 40 : 1) ergeben 2-(3-Hydroxy-pyrid-2'-yl)-4-methyl-2-thiazolin-carbonsäuremethylester als blaßgelbes Öl.

DC: Rf 0,79 in Choroform/Methanol 40 : 1.

UV (Ethanol): 210 (4,22), 238 (3,82), 313 (4,01).

IR ($CHCl_3$): 1735, 1590, 1450, 980.

$^1$H-NMR ($CHCl_3$): $\delta$ = 1,68 (s; 3H), 3,18 (d,J = 12 Hz; 1H), 3,77 (s; 3H), 3,80 (d, J = 12 Hz, 1H), 7,18—7,4 (m; 2H), 8,1—8,25 (m, 1H), 12,0 (br. s. OH).

MS: u. a. 252 ($M^+$, $C_{11}H_{12}N_2O_3S$, 24), 193 (100), 165 (4), 121 (14), 73 (23).

## Beispiel 4

45 mg (0,189 mMol) 2-(3'-Hydroxy-pyrid-2'-yl)-4-methyl-2-thiazolin-4-carbonsäure werden bei Raumtemperatur in Methanol/Wasser bis zur bleibenden Gelbfärbung mit etherischer Diazomethanlösung behandelt. Eindampfen im Vakuum, Ausschütteln mit $CHCl_3$ aus $H_2O$ und Chromatographieren an Sephadex LH 20 mit Chloroform/Methanol 1 : 1 liefern (2-(3'-Methoxy-pyrid-2'-yl)-4-methyl-2-thiazolin-4-carbonsäuremethylester als farbloses Öl.

DC: Rf 0,83 in Chloroform/Methanol/Wasser 65 : 25 : 4.

IR ($CHCl_3$); 1730, 1600, 1580, 1465, 1430, 960.

$^1$H-NMR ($CDCl_3$): $\delta$ = 1,66 (s; 3H), 3, 17 (d, J = 12 Hz, 1H), 3,74 (d, J = 12 Hz; 1H), 3,77 (s; 3H), 3,88 (s; 3H), 7,32 (d; J = 3 Hz, 2H), 8,23 (t, J = 3 Hz; 1H).

MS: u. a. 266 ($M^+$, $C_{12}H_{14}N_2O_3S$, 10), 219 (12), 207 (100), 192 (25), 166 (10), 135 (28), 73 (22).

Beispiel 5

Der Stamm Streptomyces antibioticus Tü 1998 (DSM 1865) wird während 6 Tagen bei 28° C auf einem Agarmedium der folgenden Zusammensetzung gezogen:

| | |
|---|---|
| Hefe-Extrakt (Difco) | 4 g/l |
| Malz-Extrakt (Difco) | 5 g/l |
| Glucose | 4 g/l |
| Agar (Difco) | 20 g/l |

Ein 500 ml Erlenmeyerkolben ohne Schikane mit 100 ml einer Nährlösung bestehend aus 2% vollfettem Sojamehl und 2% Mannit, pH 7,5, wird mit der erhaltenen Schrägröhrchenkultur angeimpft und während 48 Stunden auf einer Schüttelmaschine mit 250 Umdrehungen pro Minute und 50 mm Auslenkung bei 28° C inkubiert.

Die zweite Vorkulturstufe wird im Erlenmeyerkolben von 2 l Inhalt mit 4 Schikanen durchgeführt. Diese Kolben werden mit 500 ml der oben beschriebenen Sojamehl-Mannit-Nährlösung und mit 2,5% (V/V) Impfmaterial aus der ersten Schüttelkolben-Kultur beschickt. Die Bebrütung erfolgt auf einer Schüttelmaschine mit 50 mm Auslenkung und 120 Umdrehungen pro Minute bei 28° C. Nach 48 Stunden zeigt diese Vorkultur einen pH-Wert von 6,7 und wird in einer Konzentration von 5% (V/V) für die Beimpfung eines 50 l Fermenters mit 30 l frischer Sojamehl-Mannit-Nährlösung verwendet.

Ein Kleinfermenter mit 50 l Fassungsvermögen, ausgerüstet mit 4 Schikanen und einem sechsblättrigen Rührer mit einem Durchmesser von 115 mm wird mit 30 l des oben beschriebenen flüssigen Nährmediums beschickt und bei 134° C sterilisiert. Nach Zugabe von 1,5 l Impfmaterial aus der Erlenmeyerkolben-Kultur werden während 48 Stunden die folgenden Fermentationsbedingungen eingehalten:

| | |
|---|---|
| Rührung: | 600 Umdrehungen pro Minute |
| Überdruck: | 0,5 bar |
| Belüftung: | 1 l Luft pro 1 l Kulturbrühe in der Minute |
| Temperatur: | 28° C |

Nach 48 Stunden erreicht die Kulturlösung einen pH-Wert von 6,4 und eine zentrifugierbare Zellmasse von 23% (V/V) und wird für die Beimpfung eines Produktionsfermenters eingesetzt.

Für Produktionsansätze wird ein Fermenter mit 500 l Inhalt verwendet, welcher mit 4 Schikanen und einem 6blättrigen Turbinenrührer mit 230 mm Durchmesser ausgerüstet ist. 300 l derselben Nährlösung wie in den Vorkulturen werden im Fermenter sterilisiert und mit 15 l Vorkultur aus dem Kleinfermenter beimpft. Es werden die folgenden Fermentationsbedingungen eingehalten:

Rührung: 400 Umdrehungen pro Minute.

Überdruck, Belüftung und Temperatur: analog wie im Kleinfermenter.

Nach 77 Stunden wird die maximale Produktion von 2-(3'-Hydroxy-pyrid-2'yl)-4-methyl-2-thiazolin-4-carbonsäure (Salz) erreicht, worauf die Fermentation abgebrochen wird. Zu diesem Zeitpunkt weist die Kulturlösung einen pH-Wert von 6,6 und ein zentrifugierbares Mycelvolumen (p.m.v.) von 24% (V/V) auf. Zur Aufarbeitung wird das Zellmaterial durch Filtration durch eine Filterpresse unter Zusatz von 2% Decalite als Filterhilfsmittel abgetrennt und mit wenig Wasser nachgewaschen. Aus 2 Parallelansätzen werden 630 l Kulturfiltrat erhalten.

600 l bestehend aus erhaltenem Kulturfiltrat und einer Waschlösung werden mit 600 g Eisen(III)-chlorid Hexahydrat versetzt und nach Einstellung des pH-Wertes auf 7,0 im Zirkularverdampfer bei reduziertem Druck auf ein Volumen von 70 l konzentriert. Nach Ansäuern auf pH 2,5 wird ein braunrot gefärbter Niederschlag durch Filtration abgetrennt. Das klare Filtrat wird auf eine Säule gegeben, welche 70 l des makroretikulären Harzes XAD-2 (Firma ROHM und HAAS) enthielt. Nach Adsorption mit einer Durchflußgeschwindigkeit von 70 l/Std. wird mit 140 l deionisiertem Wasser nachgewaschen. Die Waschung und die nachfolgende Elution mit 50%igem wässerigem 2-Propanol erfolgen mit einer auf 100 l/Std. erhöhten Durchflußgeschwindigkeit. Die ersten 60 l eines praktisch farblosen Eluats werden verworfen. 220 l Eluat mit rotbrauner Färbung werden bei reduziertem Druck konzentriert und lyophilisiert, wobei 22,5 g Rückstand erhalten werden.

Der obige Rückstand und der abfiltrierte Niederschlag (166 g) werden in 3 Portionen in Imolarem Phosphatpuffer mit pH 8,8 gelöst (total 4 l). Der pH-Wert wird durch Zugabe von 2 N Natronlauge auf 8,0 gestellt. Nach Sättigung mit Kochsalz wird mehrmals mit Methylenchlorid extrahiert, bis die wässerige Phase keine rote Färbung mehr enthält. Die organische Phase wird mit gesättigter Kochsalzlösung gewaschen und am Rotationsverdampfer zur Trockene eingedampft.

Der erhaltene durchkristallisierte Rückstand (56,6 g) wird in 350 ml Methanol gelöst und auf eine mit 1500 g Sephadex LH-20 in Methanol gepackte Säule (7 × 160 cm) gegeben. Die Elution erfolgt mit Methanol mit einer Durchflußgeschwindigkeit von 1 l/Std. Im ersten Eluatvolumen von 3 l werden 27,9 g gelber Feststoff abgetrennt, die folgenden 0,65 l enthalten 6,05 g farbloses Öl. Die anschließenden 5 Fraktionen von je 0,25 l enthalten Ferrithiocin, welches mit einem öligen Nebenprodukt verunreinigt ist. Reines Ferrithiocin wird in den folgenden 10 Fraktionen von total 2,5 l eluiert und nach dem Eindampfen in kristalliner Form erhalten. Die weitere Reinigung der mit Öl verunreinigten Fraktionen ergibt zusätzlich dünnschichtchromatographisch einheitliches Ferrithiocin.

Für die dünnschichtchromatographische Charakterisierung werden MERCK Kieselgelplatten mit 0,25 mm Schichtdicke verwendet. Laufmittelsysteme: Chloroform/Methanol 4 : 1; Chloroform/Methanol/Wasser 130 : 50 : 8. Nachweis der roten Eigenfärbung, im UV-Licht oder durch Anfärbung mit Jod.

1 g reines Ferrithiocin wird mit 30 ml eisgekühlter 1 N Natronlauge versetzt. Das ausgeschiedene Eisenhydroxid wird nach Zugabe von Filterhilfsmittel (Hyflo) durch Filtration abgetrennt und mit wenig Wasser nachgewaschen. Das Filtrat wird bei 0° durch vorsichtige Zugabe von conc. Salzsäure auf einen pH-Wert von genau 3,0 eingestellt. Die ausgefallenen gelben Kristalle werden nach 1 Stunde bei 0° abfiltriert, mit wenig Eiswasser gewaschen und bei Raumtemperatur im Hochvakuum getrocknet, wobei 2-(3'-Hydroxy-pyrid-2'-yl)-4-methyl-2-thiazolin-4-carbonsäure in Form von hellgelben feinen verfilzten Nadeln erhalten werden. Die lyophilisierten Mutterlaugen werden in warmen Wasser gelöst und mit Methanol versetzt, wobei weitere 50 mg leicht rötlich gefärbtes Produkt anfallen. Die Kristalle aus der ersten Fraktion werden während 24 Std. bei Raumtemperatur über Phosphorpentoxid getrocknet: Smp. 115—166°; $[\alpha]_D^{20}$ + 32° ± 1° (c = 0,715% in Methanol).

## Beispiel 6

2,283 g (13 mMol) L-Cysteinhydrochlorid Monohydrat (FLUKA puriss.) werden unter Stickstoffspülung in 45 ml entgastem dest. Wasser gelöst und mit ca. 10 ml einer 2 N Natronlauge auf pH 8 gestellt. Hierauf gibt man unter Rühren eine Lösung von 1,2 g (10 mMol) 3-Hydroxy-picolinsäurenitril in 70 ml Methanol (MERCK p. A.) zu. Nach 1$^1/_2$ Stunden Reaktionsdauer bei Raumtemperatur unter Stickstoffatmosphäre und Lichtauschluß wird mit 10 ml 2 N Salzsäure angesäuert. Das Reaktionsgemisch wird während 4 Stunden im Kühlschrank aufbewahrt. Der ausgefallene schwach gelbe Niederschlag wird unter einer Stickstoffatmosphäre abfiltriert, mit wenig eisgekühltem 50%igem wässerigem Methanol gewaschen und im Hochvakuum unter Lichtausschluß bei Raumtemperatur getrocknet.

Das Filtrat wird im Vakuum konzentriert und lyophilisiert. Der erhaltene Rückstand wird möglichst konzentriert in 50%igem wässerigem Methanol gelöst. Beim Stehenlassen über Nacht im Kühlschrank bildet sich ein etwas stärker gelb gefärbter Niederschlag der 2-(3'-Hydroxy-pyrid-2'-yl)-2-thiazolin-4-carbonsäure, welcher in der oben beschriebenen Weise abfiltriert und getrocknet wird.

Das eingesetzte 3-Hydroxy-picolinsäurenitril wird nach einer Vorschrift von Niels Clauson-Kaas und Mitarbeitern aus Furfural hergestellt (Acta Chemica Scandinavica 19, 1965, 1147—1152).

## Beispiel 7

Die isomere 2-(3'-Hydroxy-pyrid-2'-yl)-2-thiazolin-4-carbonsäure wird durch analoge Umsetzung wie im Beispiel 6 beschrieben von 0,526 g (4,4 mMol) 3-Hydroxy-picolinsäurenitril mit 1,0 g (5,7 mMol) D-Cysteinhydrochlorid-Monohydrat (FLUKA puriss.) erhalten.

## Beispiel 8

In analoger Weise wie im Beispiel 6 beschrieben werden 2,05 g (13 mMol) D,L-Cysteinhydrochlorid (purum, FLUKA) mit 1,2 g (10 mMol) 3-Hydroxy-picolinsäurenitril umgesetzt, wobei in guter Ausbeute die optisch inaktive 2-(3'-Hydroxy-pyrid-2'-yl)-2-thiazolin-4-carbonsäure erhalten wird.

## Beispiel 9

2-(3'-Methoxy-pyrid-2'-yl)-2-thiazolin-4-carbonsäure wird durch Umsetzung von 3-Methoxy-picolinsäurenitril mit 1,3 Mol-Äquivalenten D,L-Cysteinhydrochlorid gemäß Beispiel 8 erhalten. 3-Methoxy-picolinsäurenitril wird durch Zugabe einer ätherischen Diazomethanlösung im Überschuß zu einer methanolischen Lösung von 3-Hydroxy-picolinsäurenitril bei Raumtemperatur hergestellt. Nach Stehenlassen über Nacht wird das Reaktionsgemisch im Vakuum zur Trockene eingedampft und ohne weitere Reinigung für die obige Umsetzung verwendet.

## Beispiel 10

Analog zu Beispiel 6 wird 1 g (5,83 mMol) $\alpha$-Methyl-D,L-Cystein-Hydrochlorid mit 0,585 g (4,86 mMol) 3-Hydroxy-picolinsäurenitril umgesetzt. Die mit 2 N Salzsäure angesäuerte Reaktionslösung wird nach 1 Stunde bei Raumtemperatur bei reduziertem Druck vom Methanol befreit und auf

eine Säule mit 200 ml XAD-2 Harz der Firma ROHM und HAAS gegeben. Man eluiert nacheinander mit dest. Wasser, 10%igem wässerigem Isopropanol und 50%igem wässerigem Isopropanol. Durch Konzentration des Eluats bei reduziertem Druck und Lyophilisation des wässerigen Rückstandes erhält man 2-(3'-Hydroxy-pyrid-2'-yl)-4-methyl-2-thiazolin-4-carbonsäure.

Die Herstellung von α-Methyl-cystein ist in der Ph.D. Dissertation von John F.G. Diederich, University of Windsor, Canada (1966) beschrieben. Siehe auch R. Thiberk, J.F.G. Diederich und K.G. Rutherford, Can. J. Chem. 43, 206 (1965).

Analog erhält man, jeweils ausgehend von einem der optischen Antipoden des α-Methyl-D,L-cysteins, die durch Racematspaltung in an sich bekannter Weise zugänglich sind, (D)- bzw. (L)-2-(3'-Hydroxy-pyrid-2'-yl)-4-methyl-2-thiazolin-4-carbonsäure.

## Beispiel 11

0,24 g (1 mMol) 2-(3'-Methoxy-pyrid-2'-yl)-2-thiazolin-4-carbonsäure werden mit 0,485 g (1,5 mMol) N,N'-Dicyclohexyl-O-(2-trimethylsilylethyl)-isoharnstoff und 5 ml abs. Dioxan während 14 Stunden auf 50° C erwärmt. Nach dem Abkühlen im Kühlschrank wird der ausgefallene N,N'-Dicyclohexyl-harnstoff abfiltriert. Das Filtrat wird zur Trockene eingedampft und der Rückstand durch Chromatographie an Kieselgel unter Verwendung von Chloroform mit zunehmendem Gehalt an Methanol gereinigt. Die dünnschichtchromatographisch einheitlichen Fraktionen werden vereinigt und zur Trockene eingedampft, worauf man 2-(3'-Methoxy-pyrid-2'-yl)-2-thiazolin-4-carbonsäure-β-(trimethylsilyl)-ethylester erhält.

Den als Reagenz verwendeten N,N'-Dicyclohexyl-O-(2-trimethylsilylethyl)-isoharnstoff erhält man folgendermaßen:

Ein Gemisch von 780 mg (3,79 mMol) N,N'-Dicyclohexylcarbodiimid, 0,6 ml (4,17 mMol; 1,1 Äquivalent) 2-Trimethylsilylethanol und 60 mg Kupfer(I)chlorid wird während 105 Minuten bei Zimmertemperatur gerührt, mit 3 ml Petrolether verdünnt und über eine Säule von neutralem Aluminiumoxid (unter Nachwaschen mit Petrolether) filtriert. Nach Abdampfen des Lösungsmittels bleibt das genannte Isoharnstoffderivat als schwach grünliches Öl zurück. Eine kleine Probe dieses Materials destilliert im Kragenkolben bei ca. 125°/0,09 Torr (Badtemperatur).

## Beispiel 12

0,41 (1,2 mMol) des rohen, amorphen β-Trimethylsilylethylesters, welcher nach der im Beispiel 11 beschriebenen Methode erhalten wird, wird in 5 ml abs. Tetrahydrofuran unter Stickstoffatmosphäre gelöst und auf −78° C abgekühlt. Unter gutem Rühren versetzt man langsam mit 2,9 ml einer frisch aus n-Butyllithium und N-Isopropyl-cyclohexylamin zubereiteten Lösung von Lithium-N-isopropylcyclohexylamid (1,3 mMol) in abs. Tetrahydrofuran. Nach 10 Min. Reaktionsdauer bei −78° C gibt man 2 ml einer 30%igen Lösung (G/V) von Methyljodid in abs. Tetrahydrofuran zu und läßt die Reaktionslösung langsam auf −25° C aufwärmen. Hierauf verdünnt man mit 50 ml Methylenchlorid und 30 ml kaltem 1 M Phosphatpuffer von pH 5,0. Die abgetrennte organische Phase wird mit frischen Phosphatpuffer und mit wässeriger Kochsalzlösung gewaschen, über Natriumsulfat getrocknet und bei reduzierten Druck zur Trockene eingedampft. Nach Reinigung des amorphen Rohproduktes durch Chromatographie an einer Säule aus 100 g säuregewaschenem Kieselgel, wobei Methylenchlorid mit steigenden Zusätzen an Isopropanol für die Elution verwendet wird, erhält man 2-(3'-Methoxy-pyrid-2'-yl)-4-methyl-2-thiazolin-4-carbonsäure-(β-trimethylsilyl-ethyl)-ester.

## Beispiel 13

0,53 g (1,5 mMol) des nach Beispiel 12 erhaltenen, dünnschichtchromatographisch einheitlichen amorphen 2-(3'-Methoxy-pyrid-2'-yl)-4-methyl-2-thiazolin-4-carbonsäure-(β-trimethylsilyl-ethyl)-esters werden mit 6 ml einer 0,5 N Lösung von Tetraethylammoniumfluorid in abs. Dimethylformamid gelöst und während 1 Stunde auf 30° C erwärmt. Das Reaktionsgemisch wird hierauf mit 30 ml eines 2 M Phosphatpuffers von pH 4,0 verdünnt und auf eine mit 400 ml XAD-2 Harz beschickte Säule gegeben. Die Säule wird nacheinander mit dest. Wasser, 10%igem wässerigem Isopropanol und zuletzt mit 50%igem wässerigem Isopropanol eluiert. Der Verlauf einer Elution wird durch Messung der Leitfähigkeit und der UV-Absorption bei 254 nm in Durchflußzellen kontrolliert. Die Fraktionen, welche das gewünschte Produkt enthalten, werden bei reduziertem Druck konzentriert und lyophilisiert. Die Rückstände werden dünnschichtchromatographisch auf Kieselgelplatten analysiert (Lösungsmittel: Chloroform/Methanol/Wasser 65 : 25 : 4). Die einheitlichen Fraktionen werden in absolutem Dioxan aufgenommen und vereinigt. Nach deren Lyophilisation erhält man 2-(3'-Methoxy-pyrid-2'-yl)-4-methyl-2-thiazolin-4-carbonsäure.

Beispiel 14

Herstellung von 1000 Kapseln mit 260 mg der aktiven Ingredienzen pro Kapsel

Zusammensetzung

| | |
|---|---|
| 2-(3'-Hydroxy-pyrid-2'-yl)-4-methyl-2-thiazolin-4-carbonsäure | 260 g |
| Talk | 36 g |
| Weizenstärke | 24 g |
| Magnesiumstearat | 16 g |
| Laktose | 4 g |
| | 340 g |

Zubereitung

Die pulverförmigen Substanzen werden durch ein Sieb mit einer Maschenweite von 0,6 mm geschlagen und gründlich gemischt. Mit je 340 g dieser Mischung werden mit einer Kapselfüllmaschine Gelatine-Kapseln bereitet.

Beispiel 15

Herstellung von 1000 Kapseln enthaltend 105 mg der aktiven Stoffe pro Kapsel

Zusammensetzung

| | |
|---|---|
| 2-(3'-Hydroxy-pyrid-2'-yl)-4-methyl-2-thiazolin-4-carbonsäure | 105 g |
| Äthyl-Cellulose | 3 g |
| Stearinsäure | 3 g |
| | 111 g |

Zubereitung

Die Äthyl-Cellulose und die Stearinsäure werden in 120 ml Methylchlorid gelöst, mit dem Antibiotikum versetzt, und die Masse wird durch ein Sieb von 0,6 mm Maschenweite bei einer Temperatur von ca. 40° geschlagen, wobei das Methylenchlorid verdampft. 156 mg des erhaltenen Granulates werden in Gelatine-Kapseln zu 0,5 ml mit Hilfe einer Kapsel-Abfüllmaschine abgefüllt.

**Patentansprüche für die Vertragsstaaten BE, CH, DE, FR, IT, LI, LU, NL und SE**

1. 2-(3'-substit.-pyrid-2'-yl)-2-thiazolin-4-carbonsäure-Derivate der Formel (I)

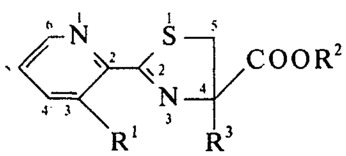

(I)

in racemischer oder optisch aktiver Form, worin $R^1$ für freies Hydroxy, Hydrocarbyloxy, Hydrocarbylcarbonyloxy oder Hydrocarbyloxycarbonyloxy steht, wobei Hydrocarbyl einen aliphatischen Rest mit 1—7 C-Atomen, einen cycloaliphatischen Rest mit 5 oder 6 Ringgliedern, einen Phenylrest oder einen durch mindestens einen solchen cycloaliphatischen Rest oder Phenylrest substituierten aliphatischen Rest mit 1—7 C-Atomen bedeutet, oder worin $R^1$ für monocyclisches fünf- oder sechsgliedriges Heterocyclyloxy mit einem Stickstoff-, Sauerstoff- oder Schwefelatom als Ringglied oder für Toluolsulfonyloxy oder aliphatisches Sulfonyloxy mit 1—7 C-Atomen steht, worin $R^2$ für Wasserstoff, einen aliphatischen Rest mit 1—7 C-Atomen, einen cycloaliphatischen Rest mit 5 oder 6 Ringgliedern, einen Phenylrest oder einen aliphatischen Rest mit 1—7 C-Atomen steht, der durch mindestens einen solchen cycloaliphatischen Rest oder Phenylrest substituiert ist, und worin $R^3$ Wasserstoff oder $C_{1-7}$-Alkyl bedeutet, und Salze dieser Verbindungen und Eisen-, Aluminium- oder Chromkomplexe solcher Verbindungen mit freier 4-Carboxy-Gruppe.

2. Verbindungen der Formel (I) gemäß Anspruch 1, worin $R_1$ freies Hydroxy, Alkoxy mit 1—7 C-Atomen, Toluolsulfonyloxy, Niederalkylsulfonyloxy, Hydrocarbylcarbonyloxy oder Hydrocarbyloxycarbonyloxy bedeutet, wobei Hydrocarbyl für Niederalkyl, Cycloalkyl mit 5 oder 6 Ringgliedern, Phenyl oder für Phenylniederalkyl steht, und worin $R^2$ einen solchen Hydrocarbylrest oder Wasserstoff und $R^3$ Wasserstoff oder Niederalkyl bedeuten, wobei das Präfix »Nieder-« einen Rest mit 1—7 C-Atomen bezeichnet, und Salze dieser Verbindungen und Eisen-, Aluminium- oder Chromkomplexe solcher Verbindungen mit freier 4-Carboxy-Gruppe.

3. Verbindungen der Formel (I) gemäß Anspruch 1, worin $R^1$ für freies Hydroxy, Niederalkoxy, Niederalkanoyloxy oder für jeweils unsubstituiertes oder durch Halogen, Niederalkyl substituiertes Phenyloxy oder Benzyloxy steht, und worin $R^2$ Wasserstoff, Niederalkyl oder einen Phenyl- oder Phenylniederalkylrest bedeutet, wobei das Präfix »Nieder-« einen Rest mit 1—7 C-Atomen bezeichnet, und Salze dieser Verbindungen und Eisen-, Aluminium- oder Chromkomplexe solcher Verbindungen mit freier 4-Carboxy-Gruppe.

4. Verbindungen der Formel (I) gemäß Anspruch 1, worin $R^1$ für freies Hydroxy oder $C_{1-7}$-Alkoxy, $R^2$ für Wasserstoff oder $C_{1-7}$-Alkyl und $R^3$ für Methyl stehen, und Salze dieser Verbindungen sowie Eisenion-Komplexe solcher Verbindungen mit freier 4-Carboxy-Gruppe.

5. Verbindungen der Formel (I) gemäß Anspruch 1, worin $R^1$ für Hydroxy oder Methoxy, $R^2$ für Wasserstoff oder Methyl und $R^3$ für Wasserstoff oder Methyl stehen, sowie deren Salze.

6. Verbindungen der Formel (I) gemäß einem der Ansprüche 1—3 und 5, worin $R^2$ für Wasserstoff steht, und deren Salze.

7. Verbindungen der Formel (I) gemäß einem der Ansprüche 1, 3 und 5—6, worin $R^3$ für Methyl steht, und deren Salze.

8. Verbindungen der Formel (I) gemäß einem der Ansprüche 1, 2 und 4, worin $R^3$ für Methyl steht, und Salze dieser Verbindungen und die in den Ansprüchen 1 oder 4 genannten Metallkomplexe solcher Verbindungen mit freier 4-Carboxy-Gruppe.

9. Verbindungen der Formel (I) gemäß einem der Ansprüche 1—3 und 5—6 worin $R^3$ für Wasserstoff steht, und deren Salze.

10. Verbindungen der Formel (I) nach einem der Ansprüche 1—9, worin $R^1$ für freies Hydroxy steht, und deren Salze.

11. Eine Verbindung gemäß Anspruch 1, nämlich 2-(3'-Hydroxy-pyrid-2'-yl)-4-methyl-2-thiazolin-4-carbonsäure.

12. Eine Verbindung gemäß Anspruch 11, nämlich die durch Fermentation von Streptomyces antibioticus (DSM 1865) erhältliche optisch aktive Form.

13. Verbindungen gemäß Anspruch 1, nämlich Salze und der Eisen(III)-komplex (Ferrithiocin) der 2-(3'-Hydroxy-pyrid-2'-yl)-4-methyl-2-thiazolin-4-carbonsäure.

14. Eine Verbindung gemäß Anspruch 1, nämlich 2-(3'-Hydroxy-pyrid-2'-yl)-4-methyl-2-thiazolin-4-carbonsäuremethylester.

15. Eine Verbindung gemäß Anspruch 1, nämlich 2-(3'-Methoxy-pyrid-2'-yl)-4-methyl-2-thiazolin-4-carbonsäuremethylester.

16. Eine Verbindung gemäß Anspruch 1, nämlich 2-(3'-Hydroxy-pyrid-2'-yl)-2-thiazolin-4-carbonsäure.

17. Das Natriumsalz einer Carbonsäure der Formel (I), worin $R^2$ für Wasserstoff steht, gemäß einem der Ansprüche 1—12 und 16.

18. Pharmazeutische Präparate, die eine Verbindung gemäß einem der Ansprüche 1—17 zusammen mit einem pharmazeutisch verwendbaren Trägermaterial enthalten.

19. Pharmazeutische Präparate zur oralen oder parenteralen Applikation zum Entzug von Schwermetallen aus dem Organismus von Warmblütern einschließlich des Menschen, die eine wirksame Dosis einer Verbindung der Formel (I) gemäß einem der Ansprüche 1—12 und 16 mit freier 4-Carboxy-Gruppe oder eines pharmazeutisch verwendbaren Salzes davon zusammen mit einem pharmazeutisch verwendbaren Trägermaterial enthalten.

20. Pharmazeutische Präparate gemäß Anspruch 19 zum Entzug von Eisen aus dem menschlichen Organismus.

21. Verwendung einer Verbindung gemäß einem der Ansprüche 1—17 zur Herstellung von Arzneimitteln.

22. Eine Verbindung der Formel (I) gemäß einem der Ansprüche 1—12 und 16 mit freier 4-Carboxy-Gruppe oder ein pharmazeutisch verwendbares Salz davon zur Verwendung als Mittel zum Entzug von Schwermetallen aus dem Organismus von Warmblütern einschließlich des Menschen durch orale oder parenterale Applikation einer wirksamen Dosis.

23. Verfahren zur Herstellung von Verbindungen gemäß Anspruch 1, dadurch gekennzeichnet, daß man

a) ein Picolinsäurederivat der Formel (II)

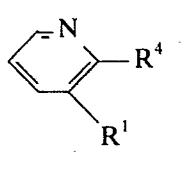

(II)

worin $R^1$ die in Anspruch 1 genannte Bedeutung hat, mit der Maßgabe, daß Hydroxy- oder andere funktionelle Gruppen gegebenenfalls in geschützter Form vorliegen, und $R^4$ eine Cyano- oder Carboxygruppe oder ein reaktives funktionelles Derivat derselben bedeuten, mit einem 2-Amino-3-mercapto-2-$R^3$-propionsäurederivat der Formel (III)

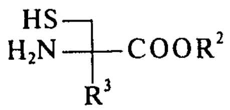

(III)

worin die Substituenten wie oben definiert sind, mit der Maßgabe, daß eine oder mehrere in der Verbindung vorhandene funktionelle Gruppen gegebenenfalls in geschützter Form vorliegen, oder mit einem reaktiven funktionellen Derivat davon umsetzt und, wenn erwünscht, gegebenenfalls vorhandene Schutzgruppen abspaltet oder

b) zur Herstellung einer Verbindung der Formel (I), worin $R^3$ für $C_{1-7}$-Alkyl steht, in einer Verbindung der Formel (I), worin $R^3$ für Wasserstoff steht und worin $R^1$ und $R^2$ wie für Formel (I) definiert sind mit der Maßgabe, daß darin vorhandene funktionelle Gruppen, wenn erwünscht, in geschützter Form vorliegen, mit einer Base in 4-Stellung ein Proton entfernt und das erhaltene Zwischenprodukt mit einem den Rest $R^3$ übertragenden Alkylierungsmittel umsetzt, in dem das Alkylkohlenstoffatom, das die neue Bindung eingeht, eine positive Partialladung trägt, und, wenn erwünscht, gegebenenfalls vorhandene Schutzgruppen abspaltet oder

c) zur Herstellung einer Verbindung der Formel (I), worin $R^1$ freies Hydroxy und/oder $R^2$ Wasserstoff bedeuten, in einer Verbindung der Formel (I), worin $R^1$ eine geschützte Hydroxygruppe und/oder $COOR^2$ eine geschützte Carboxygruppe bedeutet, mindestens eine Schutzgruppe abspaltet oder

d) zur Herstellung einer Verbindung der Formel (I), worin $R^1$ Hydroxy, $R^2$ Wasserstoff und $R^3$ Methyl bedeuten, den Stamm Streptomyces antibioticus Waksman et Henrici Tü 1998 (DSM 1865) oder eine 2-(3'-Hydroxypyrid-2'-yl)-4-methyl-2-thiazolin-4-carbonsäure bildende Mutante dieses Stammes in einer wässerigen, eine Kohlenstoff- und Stickstoffquelle sowie anorganische Salze enthaltenden Nährlösung aerob züchtet und aus der Nährlösung die 2-(3'-Hydroxy-pyrid-2'-yl)-4-methyl-2-thiazolin-4-carbonsäure oder einen stabilen Schwermetallkomplex dieser Säure isoliert und, wenn erwünscht, aus dem Schwermetallkomplex die 2-(3'-Hydroxy-pyrid-2'-yl)-4-methyl-2-thiazolin-4-carbonsäure oder ein Salz derselben freisetzt, und, wenn erwünscht, eine Verbindung der Formel (I) gemäß Anspruch 1 überführt, daß man in einer Verbindung der Formel (I) eine 3'-Hydroxy-Gruppe verestert oder verethert oder eine veresterte oder veretherte 3'-Hydroxy-Gruppe in eine freie überführt und/oder eine 4-Carboxy-Gruppe mit einem den Rest $R^2$ enthaltenden Veresterungsmittel verestert oder eine veresterte 4-Carboxy-Gruppe verseift und/oder die Verbindungen der Formel (I) in ihre Säureadditionssalze oder die Verbindungen der Formel (I) mit freier 4-Carboxy-Gruppe in ihre Metallsalze oder in die Eisen-, Aluminium- oder Chromkomplexe überführt oder racemische Gemische in die optisch aktiven Formen auftrennt.

**Patentansprüche für den Vertragsstaat AT**

1. Verfahren zur Herstellung von 2-(3'-substit.-pyrid-2'-yl)-2-thiazolin-4-carbonsäure-Derivaten der Formel (I)

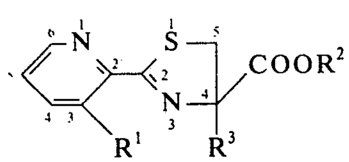

(I)

in racemischer oder optisch aktiver Form, worin $R^1$ für freies Hydroxy, Hydrocarbyloxy, Hydrocarbylcarbonyloxy oder Hydrocarbyloxycarbonyloxy steht, wobei Hydrocarbyl einen aliphatischen Rest mit 1—7 C-Atomen, einen cycloaliphatischen Rest mit 5 oder 6 Ringgliedern, einen Phenylrest oder einen durch mindestens einen solchen cycloaliphatischen Rest oder Phenylrest substituierten aliphatischen Rest mit 1—7 C-Atomen bedeutet, oder worin $R^1$ für monocyclisches fünf- oder sechsgliedriges Heterocyclyoxy mit einem Stickstoff-, Sauerstoff- oder Schwefelatom als Ringglied oder für Toluolsulfonyloxy oder aliphatisches Sulfonyloxy mit 1—7 C-Atomen steht, worin $R^2$ für Wasserstoff, einen aliphatischen Rest mit 1—7 C-Atomen, einen cycloaliphatischen Rest mit 5 oder 6 Ringgliedern, einen Phenylrest oder einen aliphatischen Rest mit 1—7 C-Atomen steht, der durch mindestens einen solchen cycloaliphatischen Rest oder Phenylrest substituiert ist, und worin $R^3$ Wasserstoff oder $C_{1-7}$-Alkyl bedeutet, oder von Salzen dieser Verbindungen oder Eisen-, Aluminium- oder Chromkomplexen solcher Verbindungen mit freier 4-Carboxy-Gruppe, dadurch gekennzeichnet, daß man

a) ein Picolinsäurederivat der Formel (II)

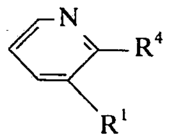

(II)

worin $R^1$ die obengenannte Bedeutung hat mit der Maßgabe, daß Hydroxy- oder andere funktionelle Gruppen gegebenenfalls in geschützer Form vorliegen, und $R^4$ eine Cyano- oder Carboxygruppe oder ein reaktives funktionelles Derivat derselben bedeuten, mit einem 2-Amino-3-mercapto-2-$R^3$-propionsäurederivat der Formel (III)

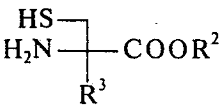

(III)

worin die Substituenten wie oben definiert sind, mit der Maßgabe, daß eine oder mehrere in der Verbindung vorhandene funktionelle Gruppen gegebenenfalls in geschützter Form vorliegen, oder mit einem reaktiven funktionellen Derivat davon umsetzt und, wenn erwünscht, gegebenenfalls vorhandene Schutzgruppen abspaltet oder

b) zur Herstellung einer Verbindung der Formel (I), worin $R^3$ für $C_{1-7}$-Alkyl steht, in einer Verbindung der Formel (I), worin $R^3$ für Wasserstoff steht und worin $R^1$ und $R^2$ wie für Formel (I) definiert sind mit der Maßgabe, daß darin vorhandene funktionelle Gruppen, wenn erwünscht, in geschützter Form vorliegen, mit einer Base in 4-Stellung ein Proton entfernt und das erhaltene Zwischenprodukt mit einem den Rest $R^3$ übertragenden Alkylierungsmittel umsetzt, in dem das Alkylkohlenstoffatom, das die neue Bindung eingeht, eine positive Partialladung trägt, und , wenn erwünscht, gegebenenfalls vorhandene Schutzgruppen abspaltet oder

c) zur Herstellung einer Verbindung der Formel (I), worin $R^1$ freies Hydroxy und/oder $R^2$ Wasserstoff bedeuten, in einer Verbindung der Formel (I), worin $R^1$ eine geschützte Hydroxygruppe und/oder $COOR^2$ eine geschützte Carboxygruppe bedeutet, mindestens eine Schutzgruppe abspaltet oder

d) zur Herstellung einer Verbindung der Formel (I), worin $R^1$ Hydroxy, $R^2$ Wasserstoff und $R^3$ Methyl bedeuten, den Stamm Streptomyces antibioticus Waksman et Henrici Tü 1998 (DSM 1865) oder eine 2-(3'-Hydroxy-pyrid-2'-yl)-4-methyl-2-thiazolin-4-carbonsäure bildende Mutante dieses Stammes in einer wässrigen, eine Kohlenstoff- und Stickstoffquelle sowie anorganische Salze enthaltenden Nährlösung aerob züchtet und aus der Nährlösung die 2-(3'-Hydroxy-pyrid-2'-yl)-4-methyl-2-thiazolin-4-carbonsäure oder einen stabilen Schwermetallkomplex dieser Säure isoliert und, wenn erwünscht, aus dem Schwermetallkomplex die 2-(3'-Hydroxy-pyrid-2'-yl)-4-methyl-2-thiazolin-4-carbonsäure oder ein Salz derselben freisetzt, und, wenn erwünscht, eine Verbindung der oben definierten Formel (I), dadurch in eine andere Verbindung der oben definierten Formel (I) überführt, daß man in einer Verbindung der Formel (I) eine 3'-Hydroxy-Gruppe verestert oder verethert oder eine veresterte oder veretherte 3'-Hydroxy-Gruppe in eine freie überführt und/oder eine 4-Carboxy-Gruppe mit einem den Rest $R^2$ enthaltenden Veresterungsmittel verestert oder eine veresterte 4-Carboxy-Gruppe verseift und/oder die Verbindungen der Formel (I) in ihre Säureadditionssalze oder die Verbindungen der Formel (I) mit freier 4-Carboxy-Gruppe in ihre Metallsalze oder in die Eisen-, Aluminium- oder Chrom komplexe überführt oder racemische Gemische in die optisch aktiven Formen auftrennt.

2. Verfahren nach Anspruch 1a), dadurch gekennzeichnet, daß man ein Picolinsäurederivat der Formel (II), worin $R^4$ für Cyano steht, mit einem Propionsäurederivat der Formel (III), worin die Mercaptogruppe und die Aminogruppe in freier Form vorliegen, umsetzt.

3. Verfahren nach Anspruch 1b), dadurch gekennzeichnet, daß man als Base ein sterisch gehindertes am Stickstoff substituiertes Alkalimetallamid verwendet.

4. Verfahren nach Anspruch 1b), dadurch gekennzeichnet, daß man als Base eine Alkalimetallniederalkylverbindung, ein Alkalimetallhydrid oder ein unsubstituiertes Alkalimetallamid verwendet.

5. Verfahren nach einem der Ansprüche 1b), 3 und 4, dadurch gekennzeichnet, daß man die Metallierungsreaktion in einem inerten aprotischen Lösungsmittel bei einer Temperatur zwischen $-100°C$ und Raumtemperatur durchführt.

6. Verfahren nach einem der Ansprüche 1b) und 3—5, dadurch gekennzeichnet, daß man als Alkylierungsmittel Niederalkylchloride, -bromide oder -iodide oder Sulfonsäure- oder Schwefelsäureester von Niederalkanolen verwendet.

7. Verfahren nach einem der Ansprüche 1—6, dadurch gekennzeichnet, daß man die Ausgangsverbindungen so wählt, daß man Verbindungen der Formel (I) gemäß Anspruch 1, worin $R^1$ freies Hydroxy, Alkoxy mit 1—7 C-Atomen, Toluolsulfonyloxy, Niederalkylsulfonyloxy, Hydrocarbylcarbonyloxy oder Hydrocarbyloxycarbonyloxy bedeutet, wobei Hydrocarbyl für Niederalkyl, Cycloalkyl mit 5 oder 6 Ringgliedern, Phenyl oder für Phenylniederalkyl steht, und worin $R^2$ einen solchen Hydrocarbylrest oder Wasserstoff und $R^3$ Wasserstoff oder Niederalkyl bedeuten, wobei das Präfix »Nieder-« einen Rest mit 1—7 C-Atomen bezeichnet, oder Salze dieser Verbindungen oder Eisen-, Aluminium- oder Chromkomplexe solcher Verbindungen mit freier 4-Carboxy-Gruppe erhält.

8. Verfahren nach einem der Ansprüche 1—6, dadurch gekennzeichnet, daß man die Ausgangsverbindungen so wählt, daß man Verbindungen der Formel (I) gemäß Anspruch 1, worin $R^1$ für freies Hydroxy, Niederalkoxy, Niederalkanoyloxy oder für jeweils unsubstituiertes oder durch Halogen, Niederalkyl oder Niederalkoxy substituiertes Phenyloxy oder Benzyloxy steht, und worin $R^2$ Wasserstoff, Niederalkyl oder einen Phenyl- der Phenylniederalkylrest bedeutet, wobei das Präfix »Nieder-« einen Rest mit 1—7 C-Atomen bezeichnet, oder Salze dieser Verbindungen oder Eisen-, Aluminium- oder Chromkomplexe solcher Verbindungen mit freier 4-Carboxy-Gruppe erhält.

9. Verfahren nach einem der Ansprüche 1—6, dadurch gekennzeichnet, daß man die Ausgangsverbindungen so wählt, daß man Verbindungen der Formel (I) gemäß Anspruch 1, worin $R^1$ für freies Hydroxy oder $C_{1-7}$-Alkoxy, $R^2$ für Wasserstoff oder $C_{1-7}$-Alkyl und $R^3$ für Methyl stehen, oder Salze dieser Verbindungen oder Eisenion-Komplexe solcher Verbindungen mit freier 4-Carboxy-Gruppe erhält.

10. Verfahren nach Anspruch 1 zur Herstellung von 2-(3'-Hydroxy-pyrid-2'-yl)-4-methyl-2-thiazolin-4-carbonsäure-Derivaten der Formel (Ia)

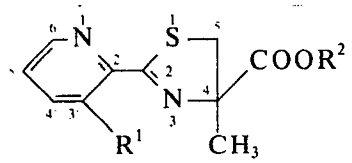

(Ia)

in racemischer oder optisch aktiver Form, worin $R^1$ für freies Hydroxy oder $C_{1-7}$-Alkoxy und $R^2$ für Wasserstoff oder $C_{1-7}$-Alkyl stehen, oder von Salzen dieser Verbindungen oder Eisenkomplexen solcher Verbindungen mit freier 4-Carboxy-Gruppe, dadurch gekennzeichnet, daß man

a) den Stamm Streptomyces antibioticus Waksman et Henrici Tü 1998 (DSM 1865) oder eine 2-(3'-Hydroxy-pyrid-2'-yl)-4-methyl-2-thiazolin-4-carbonsäure bildende Mutante dieses Stammes in einer wässerigen, eine Kohlenstoff- und Stickstoffquelle sowie anorganische Salze enthaltenden Nährlösung aerob züchtet und aus der Nährlösung die 2-(3'-Hydroxy-pyrid-2'-yl)-4-methyl-2-thiazolin-4-carbonsäure oder einen stabilen Schwermetallkomplex dieser Säure isoliert und, wenn erwünscht, aus dem Schwermetallkomplex die 2-(3'-Hydroxy-pyrid-2'-yl)-4-methyl-2-thiazolin-4-carbonsäure oder ein Salz derselben freisetzt, oder

b) ein Picolinsäurederivat der Formel (II)

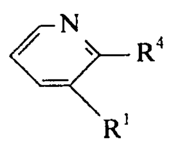

(II)

worin $R^1$ die obengenannte Bedeutung hat, wobei eine Hydroxygruppe gegebenenfalls in geschützter Form vorliegt, und $R^4$ eine Carboxy-Gruppe oder ein reaktives funktionelles Derivat derselben bedeutet, mit einem 2-Amino-3-mercapto-2-methyl-propionsäurederivat der Formel

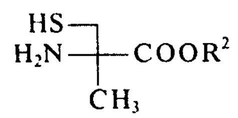

(III)

worin $R^2$ die obengenannte Bedeutung hat, wobei freies Carboxy $COOR^2$ gegebenenfalls in geschützter Form vorliegt, umsetzt, und gegebenenfalls vorhandene Schutzgruppen abspaltet, oder

c) in einer Verbindung der Formel

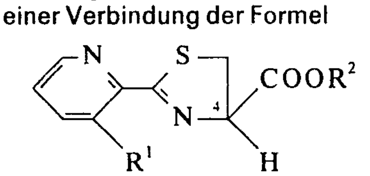

worin $R^1$ und $R^2$ die obengenannten Bedeutungen haben, wobei freies Hydroxy $R^1$ und freies Carboxy $COOR^2$ gegebenenfalls in geschützter Form vorliegen, mit einer Base in 4-Stellung ein Proton entfernt und das erhaltene Carbanion mit einem Methylierungsmittel umsetzt, in dem das Methylkohlenstoffatom eine positive Partialladung trägt, und, wenn erwünscht, eine Verbindung der oben definierten Formel (Ia) dadurch in eine andere Verbindung der oben definierten Formel (Ia) überführt, daß man in einer Verbindung der Formel (Ia) eine 3'-Hydroxy-Gruppe verethert oder eine veretherte 3'-Hydroxy-Gruppe in eine freie überführt und/oder eine Carboxygruppe mit einem den Rest $R^2$ enthaltenden Veresterungsmittel verestert oder eine veresterte Carboxygruppe verseift und/oder die Verbindungen der Formel (Ia) in ihre Säureadditionssalze oder die Verbindungen der Formel (Ia) mit freier 4-Carboxy-Gruppe in ihre Metallsalze oder in Eisenkomplexe überführt oder Racemate in die optisch aktiven Formen auftrennt.

11. Verfahren nach Anspruch 10, dadurch gekennzeichnet, daß man die Nährlösung bei einer Temperatur zwischen 18 und 40° C 3—7 Tage kultiviert.

12. Verfahren nach Anspruch 10, dadurch gekennzeichnet, daß man die 2-(3'-Hydroxy-pyrid-2'-yl)-4-methyl-2-thiazolin-4-carbonsäure in Form eines Eisen(III)komplexes isoliert.

13. Verfahren nach einem der Ansprüche 1—10, dadurch gekennzeichnet, daß man die Ausgangsverbindungen so wählt, daß man Verbindungen der Formel (I) gemäß Anspruch 1, worin $R^1$ für Hy droxy oder Methoxy, $R^2$ für Wasserstoff oder Methyl und $R^3$ für Wasserstoff oder Methyl stehen, oder deren Salze erhält.

14. Verfahren nach einem der Ansprüche 1—14, dadurch gekennzeichnet, daß man die Ausgangsverbindungen so wählt, daß man Verbindungen der Formel (I), worin $R^2$ für Wasserstoff steht, oder deren Salze erhält.

15. Verfahren nach einem der Ansprüche 1—14, dadurch gekennzeichnet, daß man die Ausgangsverbindungen so wählt, daß man eine Verbindung der Formel (I), worin $R^3$ für Methyl steht, oder ein Salz dieser Verbindung erhält.

16. Verfahren nach einem der Ansprüche 1, 2, 7, 8, 13 und 14, dadurch gekennzeichnet, daß man die Ausgangsverbindungen so wählt, daß man eine Verbindung der Formel (I), worin $R^3$ für Wasserstoff steht, oder ein Salz dieser Verbindung erhält.

17. Verfahren nach einem der Ansprüche 1—16, dadurch gekennzeichnet, daß man die Ausgangsverbindungen so wählt, daß man eine Verbindung der Formel (I), worin $R^1$ für freies Hydroxy steht, oder ein Salz dieser Verbindung erhält.

18. Verfahren nach einem der Ansprüche 1—6 und 10—12, dadurch gekennzeichnet, daß man die Ausgangsverbindungen so wählt, daß man 2-(3'-Hydroxy-pyrid-2'-yl)-4-methyl-2-thiazolin-4-carbonsäure erhält.

19. Verfahren nach einem der Ansprüche 10—12, dadurch gekennzeichnet, daß man die Ausgangsverbindungen so wählt, daß man die nach dem mikrobiologischen Verfahren erhaltene optisch aktive Form der 2-(3'-Hydroxy-pyrid-2'-yl)-4-methyl-2-thiazolin-4-carbonsäure erhält.

20. Verfahren nach einem der Ansprüche 10—12, dadurch gekennzeichnet, daß man die Ausgangsverbindungen so wählt, daß man ein Salz oder ein Eisen(III)komplex (Ferrithiocin) der 2-(3'-Hydroxy-pyrid-2'-yl)-4-methyl-2-thiazolin-4-carbonsäure erhält.

21. Verfahren nach einem der Ansprüche 1—6 und 10—12, dadurch gekennzeichnet, daß man die Ausgangsverbindungen so wählt, daß man 2-(3'-Hydroxy-pyrid-2'-yl)-4-methyl-2-thiazolin-4-carbonsäuremethylester erhält.

22. Verfahren nach einem der Ansprüche 1—6 und 10—12, dadurch gekennzeichnet, daß man die Ausgangsverbindungen so wählt, daß man 2-(3'-Methoxy-pyrid-2'-yl)-4-methyl-2-thiazolin-4-carbonsäuremethylester erhält.

23. Verfahren nach einem der Ansprüche 1, 2 und 10, dadurch gekennzeichnet, daß man die Ausgangsverbindungen so wählt, daß man 2-(3'-Hydroxy-pyrid-2'-yl)-2-thiazolin-4-carbonsäure erhält.

24. Verfahren nach einem der Ansprüche 1—17, 20 und 23, dadurch gekennzeichnet, daß man die Ausgangsverbindungen so wählt, daß man das Natriumsalz einer Carbonsäure der Formel (I), worin $R^2$ für Wasserstoff steht, erhält.

**Claims for the Contracting States: BE, CH, DE, FR, IT, LI, LU, NL, SE**

1. A 2-(3'-substituted-pyrid-2'-yl)-2-thiazoline-4-carboxylic acid derivative of the formula (I)

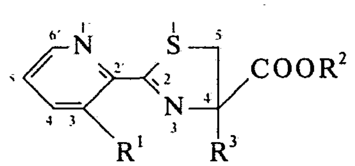

(I)

in racemic or optically active form, wherein $R^1$ ist free hydroxy, hydrocarbyloxy, hydrocarbylcarbonyloxy or hydrocarbyloxycarbonyloxy, with hydrocarbyl denoting an aliphatic radical containing 1 to 7 carbon atoms, a cycloaliphatic radical containing 5 or 6 ring members, a phenyl radical, or an aliphatic radical containing 1 to 7 carbon atoms which is substituted by at least one such cycloaliphatic radical or by at least one phenyl radical, or wherein $R^1$ is monocyclic 5- or 6-membered heterocyclyloxy containing a nitrogen, oxygen or sulfur atom as ring member, or is toluenesulfonyloxy or aliphatic sulfonyloxy containing 1 to 7 carbon atoms, $R^2$ ist hydrogen, an aliphatic radical containing 1 to 7 carbon atoms, a cycloaliphatic radical containing 5 or 6 ring members, a phenyl radical, or an aliphatic radical containing 1 to 7 carbon atoms which is substituted by at least one such cycloaliphatic radical or by at least one phenyl radical, and $R^3$ ist hydrogen or $C_1$—$C_7$alkyl, or a salt thereof or an iron, aluminium or chromium complex of such a compound containing a free 4-carboxyl group.

2. A compound of formula (I) according to claim 1, wherein $R^1$ is free hydroxy, $C_1$—$C_7$alkoxy, toluenesulfonyloxy, lower alkylsulfonyloxy, hydrocarbylcarbonyloxy or hydrocarbyloxycarbonyloxy,

with hydrocarbyl denoting lower alkyl, cycloalkyl containing 5 or 6 ring members, phenyl or phenyl-lower alkyl, and wherein $R^2$ ist such a hydrocarbyl radical or hydrogen, and $R^3$ is hydrogen or lower alkyl, with the term »lower« denoting a radical containing 1 to 7 carbon atoms, or a salt thereof or an iron, aluminium or chromium complex of such a compound containing a free 4-carboxyl group.

3. A compound of formula (I) according to claim 1, wherein $R^1$ is free hydroxy, lower alkoxy, lower alkanoyloxy, or is phenyloxy or benzyloxy, each unsubstituted or substituted by halogen, lower alkyl or lower alkoxy, and wherein $R^2$ is hydrogen, lower alkyl or a phenyl or phenyl-lower alkyl radical, with the term »lower« denoting a radical containing 1 to 7 carbon atoms, or a salt thereof or an iron, aluminium or chromium complex of such a compound containing a free 4-carboxyl group.

4. A compound of formula (I) according to claim 1, wherein $R^1$ is free hydroxy or $C_1-C_7$alkoxy, $R^2$ is hydrogen or $C_1-C_7$alkyl, and $R^3$ is methyl, or a salt thereof or an iron ion complex of such a compound containing a free 4-carboxyl group.

5. A compound of formula (I) according to claim 1, wherein $R^1$ is hydroxy or methoxy, $R^2$ is hydrogen or methyl, and $R^3$ is hydrogen or methyl, or a salt thereof.

6. A compound of formula (I) according to any one of claims 1 to 3, wherein $R^2$ is hydrogen, or a salt thereof.

7. A compound of formula (I) according to any one of claims 1, 3, 5 and 6, wherein $R^3$ is methyl, or a salt thereof.

8. A compound of formula (I) according to any one of claims 1, 2 and 4, wherein $R^3$ is methyl, or a salt thereof or a metal complex of such a compound containing a free 4-carboxylic group according to either claim 1 or claim 4.

9. A compound of formula (I) according to any one of claims 1 to 3 and 5 and 6, wherein $R^3$ is hydrogen, or a salt thereof.

10. A compound of formula (I) according to any one of claims 1 to 9, wherein $R^1$ is free hydroxy, or a salt thereof.

11. 2-(3'-Hydroxypyrid-2'-yl)-4-methyl-2-thiazoline-4-carboxylic acid according to claim 1.

12. A compound according to claim 11, namely the optically active form obtainable by fermentation of Streptomyces antibioticus (DSM 1865).

13. A compound according to claim 1, namely a salt or the iron(III) complex (ferrithiocin) of 2-(3'-hydroxypyrid-2'-yl)-4-methyl-2-thiazoline-4-carboxylic acid.

14. Methyl 2-(3'-hydroxypyrid-2'-yl)-4-methyl-2-thiazoline-4-carboxylate according to claim 1.

15. Methyl 2-(3'-methoxypyrid-2'-yl)-4-methyl-2-thiazoline-4-carboxylate according to claim 1.

16. 2-(3'-Hydroxypyrid-2'-yl)-2-thiazoline-4-carboxylic acid according to claim 1.

17. The sodium salt of a carboxylic acid of formula (I), wherein $R^2$ is hydrogen, according to any one of claims 1 to 12 and 16.

18. A pharmaceutical composition containing a compound according to any one of claims 1 to 17, together with a pharmaceutically acceptable carrier.

19. A pharmaceutical composition for oral or parenteral administration for the removal of heavy metals from the organism of warm-blooded animals, including man, which composition contains an effective dose of a compound of formula (I) containing a free 4-carboxylic group according to any one of claims 1 to 12 and 16, or of a pharmaceutically acceptable salt thereof, together with a pharmaceutically acceptable carrier.

20. A pharmaceutical composition according to claim 19 for the removal of iron from the human organism.

21. Use of a compound according to any one of claims 1 to 17 for the preparation of a pharmaceutical composition.

22. Use of a compound of formula (I) containing a free 4-carboxyl group according to any one of claims 1 to 12 and 16, or of a pharmaceutically acceptable salt thereof, for removing heavy metals from the organism of warm-blooded animals, including man, by administering an effective dose orally or parenterally.

23. A process for the preparation of a compound according to claim 1, which comprises

a) reacting a picolinic acid derivative of formula (II)

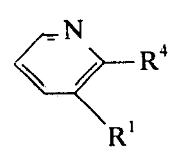

(II)

wherein $R^1$ is as defined above, with the proviso that hydroxyl or other functional groups are in unprotected or protected form, and $R^4$ is a cyano or carboxyl group, or a reactive functional derivative thereof, with a 2-amino-3-mercapto-2-$R^3$-propionic acid derivative of formula (III)

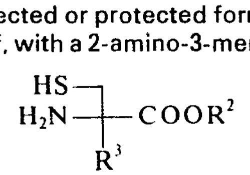

(III)

wherein the substituents are as defined above, with the proviso that one or more of the functional groups present in the compound are in unprotected or protected form, or with a reactive functional derivative thereof, and, if desired, removing any protecting groups present; or

b) to prepare a compound of formula (I), wherein $R^3$ is $C_1-C_7$alkyl, removing with a base in 4-position a proton from a compound of formula (I), wherein $R^3$ is hydrogen and $R^1$ and $R^2$ are as defined for formula (I), with the proviso that functional groups present therein, if desired, are in protected form, and reacting the resultant intermediate with an alkylating agent which introduces the radical $R^3$, and in the alkyl moiety of said alkylating agent the carbon atom which forms the novel bond carries a positive partial charge, and, if desired, removing any protecting groups present; or

c) to prepare a compound of formula (I), wherein $R^1$ is free hydroxy and/or $R^2$ is hydrogen, removing at least one protecting group from a compound of formula (I), wherein $R^1$ is a protected hydroxyl group and/or $COOR^2$ is a protected carboxyl group; or

d) to prepare a compound of formula (I), wherein $R^1$ is hydroxy, $R^2$ is hydrogen and $R^3$ is methyl, aerobically cultivating the strain Streptomyces antibioticus Waksman et Henrici Tü 1998 (DSM 1865), or a mutant of this strain which forms a 2-(3'-hydroxypyrid-2'-yl)-4-methyl-2-thiazoline-4-carboxylic acid, in an aqueous nutrient solution containing a carbon and nitrogen source as well as inorganic salts, and isolating from said nutrient solution the 2-(3'-hydroxypyrid-2'-yl)-4-methyl-2-thiazoline-4-carboxyl ic acid, or a stable heavy metal complex of said acid, and, if desired, liberating from the heavy metal complex the 2-(3'-hydroxypyrid-2'-yl)-4-methyl-2-thiazoline-4-carboxylic acid, or a salt thereof; and, if desired, converting a compound of formula (I) according to claim 1 into another compound of formula (I) according to claim 1 by esterifying or etherifying in a compound of formula (I) a 3'-hydroxyl group, or by converting an esterified or etherified 3'-hydroxyl group into a free one, and/or by esterifying a 4-carboxyl group with an esterifying agent containing the radical $R^2$, or by saponifying an esterified 4-carboxyl group; and/or converting a compound of formula (I) into an acid addition salt thereof, or converting a compound of formula (I) containing a free 4-carboxyl group into a metal salt thereof or into an iron, aluminium or chromium complex thereof, or separating a racemic mixture into the optically active forms.

**Claims for the Contracting State: AT**

1. A process for the preparation of a 2-(3'-substituted-pyrid-2'-yl)-2-thiazoline-4-carboxylic acid derivate of the formula (I)

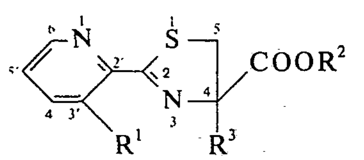

(I)

in racemic or optically active form, wherein $R^1$ is free hydroxy, hydrocarbyloxy, hydrocarbylcarbonyloxy or hydrocarbyloxycarbonyloxy, with hydrocarbyl denoting an aliphatic radial containing 1 to 7 carbon atoms, a cycloaliphatic radical containing 5 or 6 ring members, a phenyl radical, or an aliphatic radical containing 1 to 7 carbon atoms which is substituted by a least one such cycloaliphatic radical or by at least one phenyl radical, or wherein $R^1$ is monocyclic 5- or 6-membered heterocyclyloxy containing a nitrogen, oxygen or sulfur atom as ring member, or is toluenesulfonyloxy or aliphatic sulfonyloxy containing 1 to 7 carbon atoms, $R^2$ is hydrogen, an aliphatic radical containing 1 to 7 carbon atoms, a cycloaliphatic radical containing 5 or 6 ring members, a phenyl radical, or an aliphatic radical containing 1 to 7 carbon atoms which is substituted by at least one such cycloaliphatic radical or by at least one phenyl radical, and $R^3$ is hydrogen or $C_1-C_7$alkyl, or a salt thereof or an iron, aluminium or chromium complex of such a compound containing a free 4-carboxyl group, which process comprises

a) reacting a picolinic acid derivative of formule (II)

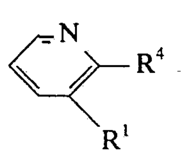

(II)

wherein $R^1$ is as defined above, with the proviso that hydroxyl or other functional groups are in unprotected or protected form, and $R^4$ is a cyano or carboxyl group, or a reactive functional derivative thereof, with a 2-amino-3-mercapto-2-$R^3$-propionic acid derivative of formula (III)

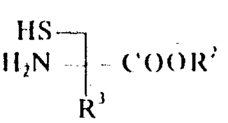

(III)

wherein the substituents are as defined above, with the proviso that one or more of the functional groups present in the compound are in unprotected or protected form, or with a reactive functional derivative thereof, and, if desired, removing any protecting groups present; or

b) to prepare a compound of formula (I), wherein $R^3$ is $C_1-C_7$alkyl, removing with a base in 4-position a proton from a compound of formula (I), wherein $R^3$ is hydrogen and $R^1$ and $R^2$ are as defined for formula (I), with the proviso that functional groups present therein, if desired, are in protected form, and reacting the resultant intermediate with an alkylating agent which introduces the radical $R^3$, and in the alkyl moiety of said alkylating agent the carbon atom which forms the novel bond carries a positive partial charge, and, if desired, removing any protecting groups present; or

c) to prepare a compound of formula (I), wherein $R^1$ is free hydroxy and/or $R^2$ is hydrogen, removing at least one protecting group from a compound of formula (I), wherein $R^1$ is a protected hydroxyl group and/or $COOR^2$ is a protected carboxyl group; or

d) to prepare a compound of formula (I), wherein $R^1$ is hydroxy, $R^2$ is hydrogen and $R^3$ is methyl, aerobically cultivating the strain Streptomyces antibioticus Waksman et Henrici Tü 1998 (DSM 1865), or a mutant of this strain which forms a 2-(3'-hydroxypyrid-2'-yl)-4-methyl-2-thiazoline-4-carboxylic acid, in an aqueous nutrient solution containing a carbon and nitrogen source as well as inorganic salts, and isolating from said nutrient solution the 2-(3'-hydroxypyrid-2'-yl)-4-methyl-2-thiazoline-4-carboxylic acid, or a stable heavy metal complex of said acid, and, if desired, liberating from the heavy metal complex the 2-(3'-hydroxypyrid-2'-yl)-4-methyl-2-thiazoline-4-carboxylic acid, or a salt thereof; and, if desired, converting a compound of formula (I) as defined above into another compound of formula (I) as defined above by esterifying or etherifying in a compound of formula (I) a 3'-hydroxyl group, or by converting an esterified or etherified 3'-hydroxyl group into a free one, and/or by esterifying a 4-carboxyl group with an esterifying agent containing the radical $R^2$, or by saponifying an esterified 4-carboxyl group; and/or converting a compound of formula (I) into acid addition salt thereof, or converting a compound of formula (I) containing a free 4-carboxyl group into a metal salt thereof or into an iron, aluminium or chromium complex thereof, or separating a racemic mixture into the optically active forms.

2. A process according to claim 1a), which comprises reacting a picolinic acid derivative of formula (II), wherein $R^4$ is cyano, with a propionic acid derivative of formula (III), wherein the mercapto group and the amino group are in free form.

3. A process according to claim 1b), wherein the base is a sterically hindered alkali metal amide substituted at the nitrogen atom.

4. A process according to claim 1b), wherein the base is an alkali metal lower alkyl compound, an alkali metal hydride or an unsubstituted alkali metal amide.

5. A process according to any one of claims 1b), 3 and 4, wherein the metallisation reaction is carried out in an inert aprotic solvent at a temperature in the range from —100° C to room temperature.

6. A process according to any one of claims 1b) and 3 to 5, wherein the alkylating agent is a lower alkyl chloride, bromide or iodide, or is a sulfonic acid ester or a sulfuric acid ester of a lower alkanol.

7. A process according to any one of claims 1 to 6, wherein the starting materials are chosen such that a compound of formula (I) is obtained, in which formula $R^1$ is free hydroxy, $C_1-C_7$alkoxy, toluenesulfonyloxy, lower alkylsulfonyloxy, hydrocarbylcarbonyloxy or hydrocarbyloxycarbonyloxy, with hydrocarbyl denoting lower alkyl, cycloalkyl containing 5 or 6 ring members, phenyl or phenyl-lower alkyl, and wherein $R^2$ is such a hydrocarbyl radical or hydrogen, and $R^3$ is hydrogen or lower alkyl, with the term »lower« denoting a radical containing 1 to 7 carbon atoms, or a salt thereof or an iron, aluminium or chromium complex of such a compound containing a free 4-carboxyl group.

8. A process according to any one of claims 1 to 6, wherein the starting materials are chosen such that a compound of formula (I) is obtained, in which formula $R^1$ is free hydroxy, lower alkoxy, lower alkanoyloxy, or is phenyloxy or benzyloxy, each unsubstituted or substituted by halogen, lower alkyl or lower alkoxy, and wherein $R^2$ is hydrogen, lower alkyl or a phenyl or phenyl-lower alkyl radical, with the term »lower« denoting a radical containing 1 to 7 carbon atoms, or a salt thereof or an iron, aluminium or chromium complex of such a compound containing a free 4-carboxyl group.

9. A process according to any one of claims 1 to 6, wherein the starting materials are chosen such that a compound of formula (I) is obtained, in which formula $R^1$ is free hydroxy of $C_1-C_7$alkoxy, $R^2$ is hydrogen or $C_1-C_7$alkyl, and $R^3$ is methyl, or a salt thereof or an iron ion complex of such a compound containing a free 4-carboxyl group.

10. A process according to claim 1 for the preparation of a 2-(3'-hydroxypyrid-2'-yl)-4-methyl-2-thiazoline-4-carboxylic acid derivative of the formula (Ia)

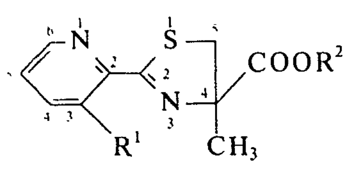

(Ia)

in racemic or optically active form, wherein $R^1$ is free hydroxy or $C_1-C_7$alkoxy, and $R^2$ is hydrogen or $C_1-C_7$alkyl, or a salt thereof or an iron complex of such a compound containing a free 4-carboxyl group, which process comprises

a) aerobically cultivating the strain Streptomyces antibioticus Waksman et Henrici Tü 1998 (DSM 1865), or a mutant of this strain which forms a 2-(3'-hydroxypyrid-2'-yl)-4-methyl-2-thiazoline-4-carboxylic acid, in an aqueous nutrient solution containing a carbon and nitrogen source as well as inorganic salts, and isolating from said nutrient solution the 2-(3'-hydroxypyrid-2'-yl)-4-methyl-2-thiazoline-4-carboxylic acid, or a stable heavy metal complex of said acid, and, if desired, liberating from the heavy metal complex the 2-(3'-hydroxypyrid-2'-yl)-4-methyl-2-thiazoline-4-carboxylic acid, or a salt thereof; or

b) reacting a picolinic acid derivative of formula (II)

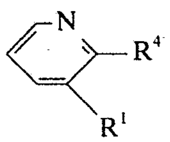

(II)

wherein $R^1$ is as defined above, with the proviso that a hydroxyl group is unprotected or protected, and $R^4$ is a carboxyl group, or a reactive functional derivative thereof, with a 2-amino-3-mercapto-2-methylpropionic acid derivative of the formula

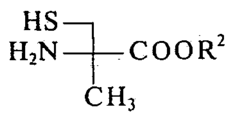

(III)

wherein $R^2$ is as defined above, with the proviso that free carboxy $COOR^2$ is in unprotected or protected form, and, if desired, removing any protecting groups present; or

c) removing with a base in 4-position a proton from a compound of the formula

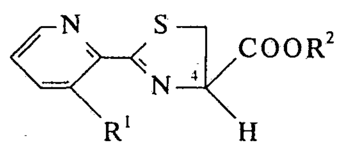

wherein $R^1$ and $R^2$ are as defined above, with the proviso that free hydroxy $R^1$ and free carboxy $COOR^2$ are in unprotected or protected form, and reacting the resultant carbanion with a methylating agent, the carbon atom of the methyl moiety of said methylating agent carrying a positive partial charge, and, if desired, converting a compound of formula (Ia) as defined above into another compound of formula (Ia) as defined above by etherifying in a compound of formula (Ia) a 3'-hydroxyl group, or by converting an etherified 3'-hydroxyl group into a free one, and/or by esterifying a carboxyl group with an esterifying agent containing the radical $R^2$, or by saponifying an esterified carboxyl group; and/or converting a compound of formula (Ia) into an acid addition salt thereof, or converting a compound of formula (Ia) containing a free 4-carboxyl group into a metal salt thereof or into an iron complex thereof, or separating a racemate into the optically active forms.

11. A process according to claim 10, wherein the nutrient solution is cultivated for 3 to 7 days at a temperature in the range from 18° to 40° C.

12. A process according to claim 10, wherein the 2-(3'-hydroxypyrid-2'-yl)-4-methyl-2-thiazoline-4-carboxylic acid is isolated in the form of the iron(III) complex.

13. A process according to any one of claims 1 to 10, wherein the starting materials are chosen such that a compound of formula (I) according to claim 1 is obtained, in which formula $R^1$ is hydroxy or methoxy, $R^2$ is hydrogen or methyl, and $R^3$ is hydrogen or methyl, or a salt thereof.

14. A process according to any one of claims 1 to 14, wherein the starting materials are chosen such that a compound of formula (I) is obtained, in which formula $R^2$ is hydrogen, or a salt thereof.

15. A process according to any one of claims 1 to 14, wherein the starting materials are chosen such that a compound of formula (I) is obtained, in which formula $R^3$ is methyl, or a salt thereof.

16. A process according to any one of claims 1, 2, 7, 8, 13 and 14, wherein the starting materials are chosen such that a compound of formula (I) is obtained, in which formula $R^3$ is hydrogen, or a salt thereof.

17. A process according to any one of claims 1 to 16, wherein the starting materials are chosen such that a compound of formula (I) is obtained, wherein $R^1$ is free hydroxy, or a salt thereof.

18. A process according to any one of claims 1 to 6 and 10 to 12, wherein the starting materials are chosen such that 2-(3'-hydroxypyrid-2'-yl)-4-methyl-2-thiazoline-4-carboxylic acid is obtained.

19. A process according to any one of claims 10 to 12, wherein the starting materials are chosen such that the optically active form of 2-(3′-hydroxypyrid-2′-yl)-4-methyl-2-thiazoline-4-carboxylic acid is obtained, said form being afforded by the microbiological process.

20. A process according to any one of claims 10 to 12, wherein the starting materials are chosen such that a salt or the iron(III) complex (ferrithiocin) of 2-(3′-hydroxypyrid-2′-yl)-4-methyl-2-thiazoline-4-carboxylic acid is obtained.

21. A process according to any one of claims 1 to 6 and 10 to 12, wherein the starting materials are chosen such that methyl (2-(3′-hydroxypyrid-2′-yl)-4-methyl-2-thiazoline-4-carboxylate is obtained.

22. A process according to any one of claims 1 to 6 and 10 to 12, wherein the starting materials are chosen such that methyl 2-(3′-methoxypyrid-2′-yl)-4-methyl-2-thiazoline-4-carboxylate is obtained.

23. A process according to any one of claims 1, 2 and 10, wherein the starting materials are chosen such that 2-(3′-hydroxypyrid-2′-yl)-2-thiazoline-4-carboxylic acid is obtained.

24. A process according to any one of claims 1 to 17, 20 and 23, wherein the starting materials are chosen such that the sodium salt of a carboxylic acid of formula (I), wherein $R^2$ is hydrogen, is obtained.

**Revendications pour les Etats contractants: BE, CH, DE, FR, IT, LI, LU, NL, SE**

1. Dérivés d'acides 2-(pyrido-2′-yl)-2-thiazoline-4-carboxyliques substitués en position 3′ du cycle pyrido, répondant à la formule 1:

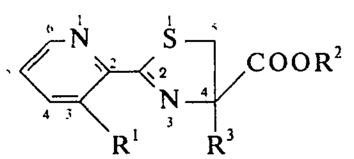

(I)

à l'état de racémiques ou d'isomères optiques, dans lesquels $R^1$ représente un groupe hydroxy libre, hydroxycarbyloxy, hydrocarbylcarbonyloxy ou hydrocarbyloxycarbonyloxy, le reste hydrocarbyle étant un reste aliphatique en C1—C7, un reste cycloaliphatique à 5 ou 6 chaînons cycliques, un reste phényle ou un reste aliphatique substitué par au moins un tel reste cycloaliphatique ou tel reste phényle, ou bien dans lesquels $R^1$ représente un groupe hétérocyclyloxy monocyclique à 5 ou 6 chaînons contenant un atome d'azote, d'oxygène ou de soufre en tant que chaînon cyclique, ou bien un groupe toluène-sulfonyloxy ou sulfonyloxy aliphatique en C1—C7, $R^2$ représente l'hydrogène, un reste aliphatique en C1—C7, un reste cycloaliphatique à 5 ou 6 chaînons cycliques, un rest phényle ou un reste aliphatique en C1—C7 substitué par au moins un tel reste cycloaliphatique ou un tel reste phényle, et $R^3$ représente l'hydrogène ou un groupe alkyle en C1—C7, et les sels de ces composés et les complexes de fer, d'aluminium ou de chrome de ces composés protant un groupe carboxyle libre en position 4.

2. Composés de formule I selon la revendication 1, dans lesquels $R^1$ représente un groupe hydroxy libre, alcoxy en C1—C7, toluène-sulfonyloxy, (alkyle inférieur)-sulfonyloxy, hydrocarbylcarbonyloxy ou hydrocarbyloxycarbonyloxy, le reste hydrocarbyle étant un reste alkyle inférieur, cycloalkyle à 5 ou 6 chanînons cycliques, phényle ou phényl-(alkyle inférieur), $R^2$ représente un tel reste hydrocarbyle ou l'hydrogène et $R^3$ représente l'hydrogène ou un groupe alkyle inférieure, l'expression »inférieur« s'appliquant à un reste en C1—C7, et les sels de ces composés et les complexes de fer, d'aluminium ou de chrome de ces composés qui portent un groupe carboxyle libre en position 4.

3. Composés de formule I selin la revendication 1, dans lesquels $R^1$ représente un groupe hydroxy libre, alcoxy inférieur, alcanoyloxy inférieur ou phényloxy ou benzyloxy ces deux derniers non substitués ou substitués par des halogènes, des groupes alkyle inférieurs ou alcoxy inférieurs, $R^2$ représente l'hydrogène, un groupe alkyle inférieur, phényle ou phényl-(alkyle inférieur), l'expression »inférieur« s'appliquant à un reste en C1—C7, et les sels de ces composés et les complexes de fer, d'aluminium ou de chrome de ces composés qui portent un groupe carboxyle libre en position 4.

4. Composés de formule I selon la revendication 1, dans lesquels $R^1$ représente un groupe hydroxy libre ou alcoxy en C1—C7, $R^2$ représente l'hydrogène ou un groupe alkyle en C1—C7 et $R^3$ un groupe méthyle, et les sels de ces composés et les complexes de l'ion fer de ces composés qui portent un groupe carboxyle libre en position 4.

5. Composés de formule I selon la revendication 1, dans lesquels $R^1$ représente un groupe hydroxy ou méthoxy, $R^2$ représente l'hydrogène ou un groupe méthyle et $R^3$ représente l'hydrogène ou un groupe méthyle, et leurs sels.

6. Composés de formule I selon l'une des revendications 1 à 3 et 5, dans lesquels $R^2$ représente l'hydrogène, et leurs sels.

7. Composés de formule I selon l'une des revendications 1, 3 et 5—6, dans lesquels $R^3$ représente un groupe méthyle, et leurs sels.

8. Composés de formule I selon l'une des revendications 1, 2 et 4, dans lesquels $R^3$ représente un

groupe méthyle, et les sels de ces composés, et les complexes métalliques mentionnés dans la revendication 1 ou 4 de ces composés qui portent un groupe carboxyle libre en position 4.

9. Composés de formule I selon l'une des revendications 1 à 3 et 5—6, dans lesquels $R^3$ représente l'hydrogène, et leurs sels.

10. Composés de formule I selon l'une des revendications 1 à 9, dans lesquels $R^1$ représente un groupe hydroxy libre, et leurs sels.

11. Un composé selon la revendication 1, à savoir l'acide 2-(3'-hydroxy-pyrido-2'-yl)-4-méthyl-2-thiazoline-4-carboxylique.

12. Un composé selon la revendication 11, à savoir l'isomère optique obtenu par fermentation de Streptomyces antibioticus (DSM 1865).

13. Composés selon la revendication 1, à savoir les sels et le complexe de fer-III (Ferrithiocine) de l'acide 2-(3'-hydroxy-pyrido-2'-yl)-4-méthyl-2-thiazoline-4-carboxylique.

14. Un composé selon la revendication 1, à savoir le 2-(3'-hydroxy-pyrido-2'-yl)-4-méthyl-2-thiazoline-4-carboxylate de méthyle.

15. Un composé selon la revendication 1, à savoir le 2-(3'-méthoxy-pyrido-2'-yl)-4-méthyl-2-thiazoline-4-carboxylate de méthyle.

16. Un composé selon la revendication 1, à savoir l'acide 2-(3'-hydroxy-pyrido-2'-yl)-2-thiazoline-4-carboxylique.

17. Le sel de sodium d'un acide carboxylique de formule I dans lequel $R^2$ représente l'hydrogène, selon l'une des revendications 1 à 12 et 16.

18. Compositions pharmaceutiques contenant un composé selon l'une des revendications 1 à 17, avec un véhicule acceptable pour l'usage pharmaceutique.

19. Compositions pharmaceutiques pour l'administration orale ou parentérale, pour éliminer les métaux lourds de l'organisme d'être à sang chaud, y compris l'homme, contenant une dose active d'un composé de formule I selon l'une des revendications 1 à 12 et 16, avec le groupe carboxyle libre en position 4, ou un sel d'un tel composé acceptable pour l'usage pharmaceutique, avec un véhicule acceptable pour l'usage pharmaceutique.

20. Compositions pharmaceutiques selon la revendication 19, pour l'élimination du fer de l'organisme humain.

21. Utilisation d'un composé selon l'une des revendications 1 à 17, pour la préparation de médicaments.

22. Un composé de formule I selon l'une des revendications 1 à 12 et 16, portant un groupe carboxyle libre en position 4, ou un sel d'un tel composé acceptable pour l'usage pharmaceutique, pour l'utilisation en tant qu'agent servant à éliminer les métaux lourds de l'organisme des êtres à sang chaud, y compris l'homme, par administration orale ou parentérale d'une dose active.

23. Procédé de préparation des composés selon la revendication 1, caractérisé en ce que:

a) on fait réagir un dérivé de l'acide picolique de formule II:

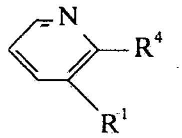

(II)

dans laquelle $R^1$ a la signification indiquée dans la revendication 1, étant spécifié que le groupe hydroxy ou les autres groupes fonctionnels sont éventuellement à l'état protégé, et $R^4$ représente un groupe cyano ou carboxy ou un dérivé fonctionnel réactif d'un même groupe, avec un dérivé de l'acide 2-amino-3-mercapto-2-$R^3$-propionique de formule III:

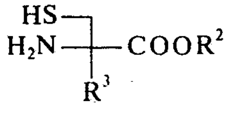

(III)

dans laquelle les symboles ont les significations indiquées ci-dessus, étant spécifié qu'un ou plusieurs des groupes fonctionnels existant dans le composé sont éventuellement à l'état protégé, ou avec un dérivé fonctionnel réactif d'un tel composé et, si on le désire, on élimine les groupes protecteurs éventuellement présents, ou bien

b) pour préparer un composé de formule I dans laquelle $R^3$ représente un groupe alkyle en C1—C7, on part d'un composé de formule I dans laquelle $R^3$ représente l'hydrogène et $R^1$ et $R^2$ ont les significations indiquées en référence à la formule I, étant spécifié que les groupes fonctionnels présents sont lorsqu'on le désire à l'état protégé, et dans ce composé, on élimine un proton en position 4 à l'aide d'une base et on fait réagir le produit intermédiaire obtenu avec un agent alkylant apportant le reste $R^3$ et dans lequel l'atome de carbone du groupe alkyle qui participe à la nouvelle liaison porte une charge partielle positive, et si on le désire, on élimine les groupes protecteurs éventuellement présents, ou bien

c) pour préparer un composé de formule I dans laquelle $R^1$ représente un groupe hydroxy libre et/ou $R^2$ l'hydrogène, on part d'un composé de formule I dans laquelle $R^1$ représente un groupe hydroxy protégé et/ou $COOR^2$ représente un groupe carboxy protégé et on élimine au moins un groupe protecteur, ou bien

d) pour préparer un composé de formule I dans laquelle $R^1$ représente un groupe hydroxy, $R^2$ l'hydrogène et $R^3$ un groupe méthyle, on soumet la souche Streptomyces antibioticus Waksman et Henrici Tü 1998 (DSM 1865) ou un mutant de cette souche formant l'acide 2-(3'-hydroxy-pyrido-2'-yl)-4-méthyl-2-thiazoline-4-carboxylique à culture aérobie dans une solution nutritive aqueuse contenant une source de carbone et une source d'azote et des sels minéraux et, à partir de la solution nutritive, on isole l'acide 2-(3'-hydroxy-pyrido-2'-yl)-4-méthyl-2-thiazoline-4-carboxylique ou un complexe de métal lourd stable de cet acide, et si on le désire, on libère à partir du complexe de métal lourd l'acide 2-(3'-hydroxy-pyrido-2'-yl)-4-méthyl-2-thiazoline-4-carboxylique ou un sel de cet acide, et si on le désire, on convertit un composé de formule I selon la revendication 1 en un autre composé de formule I selon la revendication 1 en estérifiant ou en éthérifiant dans un composé de formule I un groupe 3'-hydroxy ou en libérant un groupe 3'-hydroxy estérifié ou éthérifié et/ou en estérifiant un groupe 4-carboxy par un agent estérifiant contenant le reste $R^2$ ou en saponifiant un groupe 4-carboxy estérifié et/ou on convertit les composés de formule I en leurs sels formés par addition avec des acides ou les composés de formule I portant un groupe 4-carboxy libre en leurs sels métalliques ou en les complexes de fer, d'aluminium ou de chrome, ou on sépare les mélanges racémiques en les isomères optiques.

**Revendications pour l'Etat contractant: AT**

1. Procédé de préparation de dérivés d'acides 2-(pyrido-2'-yl)-2-thiazoline-4-carboxyliques substitués dans la position 3' du noyau pyrido, répondant à la formule I:

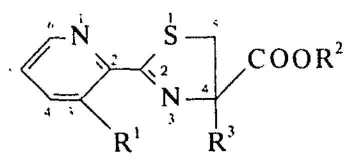

(I)

à l'état de racémiques ou d'isomères optiques, dans lesquels $R^1$ représente un groupe hydroxy libre, hydrocarbyloxy, hydrocarbylcarbonyloxy ou hydrocarbyloxycarbonyloxy, le reste hydrocarbyle étant un reste aliphatique en C1—C7, un reste cycloaliphatique à 5 ou 6 chaînons cycliques, un reste phényle ou un reste aliphatique en C1—C7 substitué par au moins un tel reste cycloaliphatique ou un tel reste phényle, ou bien $R^1$ représente un reste hétérocyclyloxy monocyclique à 5 ou 6 chaînons contenant un atome d'azote, d'oxygène ou de soufre en tant que chaînon cyclique, ou un groupe toluène-sulfonyloxy ou sulfonyloxy aliphatique en C1—C7, $R^2$ représente l'hydrogène, un reste aliphatique en C1—C7, un reste cycloaliphatique contenant 5 ou 6 chaînons cycliques, un reste phényle ou un reste aliphatique en C1—C7 substitué par au moins un tel reste cycloaliphatique ou un tel reste phényle, et $R^3$ représente l'hydrogène ou un groupe alkyle en C1—C7, ou de sels de ces composés ou de complexes de fer, d'aluminium ou de chrome de ces composés qui portent un groupe carboxyle libre en position 4, caractérisé en ce que:

a) on fait réagir un dérivé de l'acide picolique de formule II:

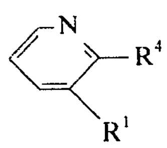

(II)

dans laquelle $R^1$ a la signification indiquée ci-dessus, étant spécifié que les groupes hydroxy ou les autres groupes fonctionnels sont éventuellement à l'état protégé et $R^4$ représente un groupe cyano ou carboxy ou un dérivé fonctionnel réactif d'un tel groupe, avec un dérivé de l'acide 2-amino-3-mercapto-2-$R^3$-propionique de formule III:

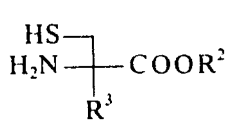

(III)

dans laquelle les symboles ont les significations indiquées ci-dessus, étant spécifié qu'un ou plusieurs des groupes fonctionnels existant dans le composé sont éventuellement à l'état protégé, ou avec un dérivé fonctionnel réactif d'un tel composé, et si on le désire, on élimine les groupes protecteurs éventuellement présents ou bien

b) pour préparer un composé de formule I dans laquelle $R^3$ représente un groupe alkyle en C1—C7, on part d'un composé de formule I dans laquelle $R^3$ représente l'hydrogène et $R^1$ et $R^2$ ont les significations indiquées en référence à la formule I, étant spécifié que, si on le désire, les groupes fonctionnels éventuellement présents sont à l'état protégé, et dans ce composé on élimine un proton en position 4 à l'aide d'une base et on fait réagir le produit intermédiaire obtenu avec un agent alkylant apportant le reste $R^3$ et dans lequel l'atome de carbone du groupe alkyle qui participe à la nouvelle liaison porte une charge partielle positive, et si on le désire, on élimine les groupes protecteurs éventuellement présents, ou bien

c) pour préparer un composé de formule I dans laquelle $R^1$ représente un groupe hydroxy libre et/ou $R^2$ l'hydrogène, on part d'un composé de formule I dans laquelle $R^1$ représente un groupe hydroxy protégé et/ou $COOR^2$ un groupe carboxy protégé, et on élimine au moins un groupe protecteur, ou bien

d) pour préparer un composé de formule I dans laquelle $R^1$ représente un groupe hydroxy, $R^2$ l'hydrogène et $R^3$ un groupe méthyle, on soumet la souche Streptomyces antibioticus Waksman et Henrici Tü 1998 (DSM 1865) ou un mutant de cette souche formant l'acide 2-(3'-hydroxypyrido-2'-yl)-4-méthyl-2-thiazoline-4-carboxylique à culture aérobie dans une solution nutritive aqueuse contenant une source de carbone et une source d'azote et des sels minéraux, et à partir de la solution nutritive, on isole l'acide 2-(3'-hydroxypyrido-2'-yl)-4-méthyl-2-thiazoline-4-carboxylique ou un complexe de métal lourd stable de cet acide, et si on le désire, à partir du complexe de métal lourd, on libère l'acide 2-(3'-hydroxypyrido-2'-yl)-4-méthyl-2-thiazoline-4-carboxylique ou un sel de cet acide, et si on le désire, on convertit un composé de formule I ci-dessus en un autre composé de formule I ci-dessus en estérifiant ou en éthérifiant dans un composé de formule I un groupe 3'-hydroxy ou en convertissant un groupe 3'-hydroxy estérifié en un groupe libre et/ou en estérifiant un groupe 4-carboxy à l'aide d'un agent estérifiant contenant le reste $R^2$ ou en saponifiant un groupe 4-carboxy estérifié, et/ou on convertit les composés de formule I en leurs sels formés par addition avec des acides ou les composés de formule I portant un groupe carboxyle libre en position 4 en leurs sels métalliques ou en les complexes de fer, d'aluminium ou de chrome, ou on sépare les mélanges racémiques en les isomères optiques.

2. Procédé selon la revendication 1a), caractérisé en ce que l'on fait réagir un dérivé de l'acide picolique de formule II dans laquelle $R^4$ représente un groupe cyano avec un dérivé de l'acide propionique de formule III dans laquelle le groupe mercapto et le groupe amino sont libres.

3. Procédé selon la revendication 1b), caractérisé en ce que l'on utilise en tant que base un amidure de métal alcalin substitué et faisant l'objet d'un empêchement stérique sur l'azote.

4. Procédé selon la revendication 1b), caractérisé en ce que l'on utilise comme base un dérivé alkyle inférieur de métal alcalin, un hydrure de métal alcalin ou un amidure de métal alcalin non substitué.

5. Procédé selon l'une des revendications 1b), 3 et 4, caractérisé en ce que la réaction de métallisation est effectuée dans un solvant aprotonique inerte à une température comprise entre —100° C et la température ambiante.

6. Procédé selon l'une des revendications 1b), et 3 à 5, caractérisé en ce que l'on utilise en tant qu'agents alkylants des chlorures, bromures ou iodures d'alkyle inférieurs ou des esters d'acides sulfoniques ou de l'acide sulfurique d'alcanols inférieurs.

7. Procédé selon l'une des revendications 1 à 6, caractérisé en ce que l'on choisit les composés de départ de manière à obtenir des composés de formule I selon la revendication 1 dans laquelle $R^1$ représente un groupe hydroxy libre, alcoxy en C1—C7, toluène-sulfonyloxy, alkylsulfonyloxy inférieur, hydrocarbylcarbonyloxy ou hydrocarbyloxycarbonyloxy, le reste hydrocarbyle étant un groupe alkyle inférieur, cycloalkyle à 5 ou 6 chaînons cycliques, phényle ou phényl-(alkyle inférieur) et $R^2$ représente un tel reste hydrocarbyle ou l'hydrogène et $R^3$ représente l'hydrogène ou un groupe alkyle inférieur, l'expression »inférieur« s'appliquant à un reste en C1—C7, ou des sels de ces composés ou des complexes de fer, d'aluminium ou de chrome de ces composés qui portent un groupe carboxyle libre en position 4.

8. Procédé selon l'une des revendications 1 à 6, caractérisé en ce que l'on choisit les composés de départ de manière à obtenir des composés de formule I selon la revendication 1 dans laquelle $R^1$ représente un groupe hydroxy libre, alcoxy inférieur, alcanoyloxy inférieur ou phényloxy ou benzyloxy, ces deux derniers non substitués ou substitués par des halogènes, des groupes alkyle inférieurs ou alcoxy inférieurs, et $R^2$ représente l'hydrogène, un groupe alkyle inférieur, phényle ou phényl-(alkyle inférieur), l'expression »inférieur« s'appliquant à un reste en C1—C7, ou des sels de ces composés ou des complexes de fer, d'aluminium ou de chrome de ces composés qui portent un groupe carboxyle libre en position 4.

9. Procédé selon l'une des revendications 1 à 6, caractérisé en ce que l'on choisit les composés de départ de manière à obtenir des composés de formule I selon la revendication 1 dans laquelle $R^1$ représente un groupe hydroxy libre ou un groupe alcoxy en C1—C7, $R^2$ représente l'hydrogène ou un groupe alkyle en C1—C7 et $R^3$ représente un groupe méthyle, ou des sels de ces composés ou des complexes de l'ion fer de ces composés qui portent un groupe carboxyle libre en position 4.

10. Procédé selon la revendication 1, pour la préparation de dérivés de l'acide 2-(3'-hydroxypyrido-2'-yl)-4-méthyl-2-thiazoline-4-carboxylique de formule Ia:

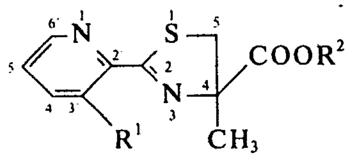

(Ia)

à l'état de racémiques ou d'isomères optiques, dans lesquels $R^1$ représente un groupe hydroxy libre ou alcoxy en C1—C7 et $R^2$ représente l'hydrogène ou un groupe alkyle en C1—C7, ou de sels de ces composés ou de complexes de fer de ces composés qui portent un groupe carboxyle libre en position 4, caractérisé en ce que:

a) on soumet la souche Streptomyces antibioticus Waksman et Henrici Tü 1998 (DSM 1865) ou un mutant de cette souche formant l'acide 2-(3'-hydroxypyrido-2'-yl)-4-méthyl-2-thiazoline-4-carboxylique à culture aérobie dans une solution nutritive aqueuse contenant une source de carbone et une source d'azote et des sels minéraux, et à partir de la solution nutritive, on isole l'acide 2-(3'-hydroxypyrido-2'-yl)-4-méthyl-2-thiazoline-4-carboxylique ou un complexe de métal lourd stable de cet acide et, si on le désire, on libère l'acide 2-(3'-hydroxypyrido-2'-yl)-4-méthyl-2-thiazoline-4-carboxylique ou un sel de cet acide à partir du complexe de métal lourd, ou bien

b) on fait réagir un dérivé de l'acide picolique répondant à la formule II:

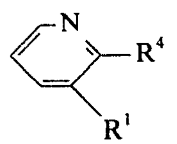

(II)

dans laquelle $R^1$ a la signification indiquée ci-dessus, un groupe hydroxy étant éventuellement protégé, et $R^4$ représente un groupe carboxy ou un dérivé fonctionnel réactif de ce groupe, avec un dérivé de l'acide 2-amino-3-mercapto-2-méthyl-propionique répondant à la formule

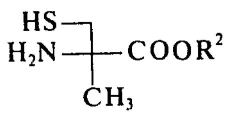

dans laquelle $R^2$ a la signification indiquée ci-dessus, un groupe carboxyle libre $COOR^2$ étant éventuellement protégé, et le cas échéant, on élimine les groupes protecteurs présents, ou bien

c) dans un composé de formule

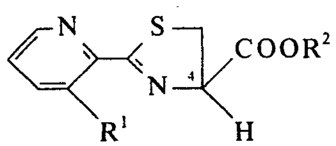

dans laquelle $R^1$ et $R^2$ ont les significations indiquées ci-dessus, un groupe hydroxy libre $R^1$ et un groupe carboxyle libre $COOR^2$ étant éventuellement protégés, et dans ce composé, on élimine un proton en position 4 à l'aide d'une base et on fait réagir le carbanion obtenu avec un agent méthylant dans lequel l'atome de carbone du groupe méthyle porte une charge partielle positive et, si on le désire, on convertit un composé de formule Ia définie ci-dessus en un autre composé de formule Ia définie ci-dessus en éthérifiant dans un composé de formule Ia un groupe 3'-hydroxy ou en convertissant un groupe 3'-hydroxy éthérifié en un groupe libre et/ou en estérifiant un groupe carboxy par un agent estérifiant contenant le reste $R^2$ ou en saponifiant un groupe carboxy estérifié et/ou on convertit les composés de formule Ia en leurs sels formés par addition avec des acides ou les composés de formule Ia contenant un groupe carboxy libre en position 4 en leurs sels métalliques ou en complexes de fer, ou on sépare les racémates en les isomères optiques.

11. Procédé selon la revendication 10, caractérisé en ce qu'on cultive la solution nutritive à une température de 18 à 40° C pendant 3 à 7 jours.

12. Procédé selon la revendication 10,caractérisé en ce que l'on isole l'acide 2-(3'-hydroxypyrido-2'-yl)-4-méthyl-2-thiazoline-4-carboxylique à l'état de complexe de fer-III.

13. Procédé selon l'une des revendications 1 à 10, caractérisé en ce que l'on choisit les composés de départ de manière à obtenir des composés de formule I selon la revendication 1 dans laquelle $R^1$ représente un groupe hydroxy ou méthoxy, $R^2$ représente l'hydrogène ou un groupe méthyle et $R^3$ l'hydrogène ou un groupe méthyle, ou leurs sels.

14. Procédé selon l'une des revendications 1 à 13, caractérisé en ce que l'on choisit les composés de départ de manière à obtenir des composés de formule I dans laquelle $R^2$ représente l'hydrogène, ou leurs sels.

15. Procédé selon l'une des revendications 1 à 14, caractérisé en ce que l'on choisit les composés de départ de manière à obtenir un composé de formule I dans laquelle $R^3$ représente un groupe méthyle ou un sel de ce composé.

16. Procédé selon l'une des revendications 1, 2, 7, 8, 13 et 14, caractérisé en ce que l'on choisit les composés de départ de manière à obtenir un composé de formule I dans laquelle $R^3$ représente l'hydrogène, ou un sel de ce composé.

17. Procédé selon l'une des revendications 1 à 6, caractérisé en ce que l'on choisit les composés de départ de manière à obtenir un composé de formule I dans laquelle $R^1$ représente un groupe hydroxy libre, ou un sel de ce composé.

18. Procédé selon l'une des revendications 1 à 6 et 10 à 12, caractérisé en ce que l'on choisit les composés de départ de manière à obtenir l'acide 2-(3'-hydroxypyrido-2'-yl)-4-méthyl-2-thiazoline-4-carboxylique.

19. Procédé selon l'une des revendications 10 à 12, caractérisé en ce que l'on choisit les composés de départ de manière à obtenir la forme énantiomère de l'acide 2-(3'-hydroxypyrido-2'-yl)-4-méthyl-2-thiazoline-4-carboxylique obtenue par le procédé microbiologique.

20. Procédé selon l'une des revendications 10 à 12, caractérisé en ce que l'on choisit les composés de départ de manière à obtenir un sel ou le complexe de fer-III (Ferrithiocine) de l'acide 2-(3'-hydroxypyrido-2'-yl)-4-méthyl-2-thiazoline-4-carboxylique.

21. Procédé selon l'une des revendications 1 à 6 et 10 à 12, caractérisé en ce que l'on choisit les composés de départ de manière à obtenir le 2-(3'-hydroxypyrido-2'-yl)-4-méthyl-2-thiazoline-4-carboxylate de méthyle.

22. Procédé selon l'une des revendications 1 à 6 et 10 à 12, caractérisé en ce que l'on choisit les composés de départ de manière à obtenir le 2-(3'-méthoxypyrido-2'-yl)-4-méthyl-2-thiazoline-4-carboxylate de méthyle.

23. Procédé selon l'une des revendications 1, 2 et 10, caractérisé en ce que l'on choisit les composés de départ de manière à obtenir l'acide 2-(3'-hydroxypyrido-2'-yl)-2-thiazoline-4-carboxylique.

24. Procédé selon l'une des revendications 1 à 17, 20 et 23, caractérisé en ce que l'on choisit les composés de départ de manière à obtenir le sel de sodium d'un acide carboxylique de formule I dans laquelle $R^2$ représente l'hydrogène.